# EUROPÄISCHE PATENTSCHRIFT

(11) **EP 3 742 171 B1**
(45) Veröffentlichungstag und Bekanntmachung des Hinweises auf die Patenterteilung: **03.04.2024**
(21) Anmeldenummer: 20174851.4
(22) Anmeldetag: 15.05.2020
(51) Int. Cl.: B01L 9/00, B01L 3/02, G01N 35/10, G01N 35/02, G01N 35/00

(54) **STEUERUNGSEINRICHTUNG FÜR PIPETTIERAUTOMATEN**
CONTROL DEVICE FOR AUTOMATIC PIPETTING DEVICES
DISPOSITIF DE COMMANDE POUR DISPOSITIFS AUTOMATIQUES DE PIPETAGE

(30) Priorität: 21.05.2019 DE 102019113531
(43) Veröffentlichungstag der Anmeldung: 25.11.2020
(73) Patentinhaber: BRAND GMBH + CO KG, 97877 Wertheim (DE)
(72) Erfinder: Berberich, Christian, 63930 Neunkirchen (DE); Scholten, David, 97906 Faulbach (DE)
(74) Vertreter: Hoffmann, Oliver

(56) Entgegenhaltungen:
- EP-A1- 3 021 123
- EP-A1- 3 171 177
- WO-A2-2017/143182
- US-A1- 2004 142 486
- US-A1- 2018 340 949

## Beschreibung

Die Erfindung betrifft eine computer-implementierte Steuerungseinrichtung zum Steuern eines Pipettierautomaten mit den Merkmalen des Oberbegriffs von Anspruch 1, einen Pipettierautomaten mit einer solchen Steuerungseinrichtung, ein Verfahren zur Steuerung eines Pipettierautomaten gemäß dem Oberbegriff von Anspruch 9, ein Computerprogrammprodukt gemäß Anspruch 16 und eine Verwendung einer Steuerungseinrichtung zur Steuerung eines Pipettierautomaten gemäß Anspruch 17.

Ein Pipettierautomat im Sinne der vorliegenden Erfindung ist vorzugsweise eine Vorrichtung, die zum vollautomatischen Flüssigkeitstransfer ausgebildet ist. Hierzu weist der Pipettierautomat vorzugsweise eine in mehrere Raumrichtungen bewegbare Pipettiervorrichtung auf. Grundsätzlich kann die vorschlagsgemäße Steuerungseinrichtung jedoch auch für sonstige Vorrichtungen, insbesondere für sonstige Laborautomaten, insbesondere zur Repositionierung von Substanzen oder Gegenständen, verwendet werden.

Ein Pipettierautomat der in Rede stehenden Art ist vorzugsweise dazu ausgebildet, eine Pipettiervorrichtung, auch Pipettierkopf oder Liquid End genannt, zwischen unterschiedlichen Betriebspositionen zu bewegen. Hierzu kann der Pipettierautomat eine Positioniereinrichtung aufweisen, insbesondere eine X-/Y-/Z-Verfahreinrichtung, durch die die Pipettiervorrichtung bewegt werden kann. Die Positioniereinrichtung kann auch ein Roboterarm sein oder aufweisen, durch den die Pipettiervorrichtung zumindest im Wesentlichen frei im Raum bewegbar ist.

Eine Pipettiervorrichtung im Sinne der vorliegenden Erfindung ist vorzugsweise ein verfahrbarer Kopf mit einer oder mehreren Verdrängereinheiten oder sonstigen Einrichtungen zur Erzeugung von Überdruck oder Unterdruck zum Ansaugen oder Ausstoßen von Flüssigkeiten. Die Pipettiervorrichtung weist vorzugsweise eine oder mehrere Koppelstellen zum Anbringen oder Auswechseln von einer oder mehreren Pipettenspitzen oder Spritzen auf. Den jeweiligen Pipettenspitzen bzw. Koppelstellen sind, vorzugsweise jeweils, Verdrängereinheiten zugeordnet. Die Spritze bzw. die jeweiligen Spritzen ist/sind vorzugsweise als Verdrängereinheit ausgebildet oder bilden solche.

Die Pipettiervorrichtung kann eine oder mehrere Pipettenspitzen oder Spritzen an einer oder mehreren Koppelstellen aufweisen, wobei bei Pipettiervorrichtungen mit lediglich einer einzigen Pipettenspitze oder Spritze von einer Einkanal-Pipettiervorrichtung und bei einer Pipettiervorrichtung mit mehreren Pipettenspitzen oder Spritzen von einer Mehrkanal-Pipettiervorrichtung die Rede ist.

Im Weiteren wird die Pipettiervorrichtung in der Ausführung mit Pipettenspitzen behandelt. Deren Verdrängereinheit(en) weist/weisen meist eine oder mehrere Zylinder-Kolben-Anordnungen auf, die dazu ausgebildet ist, Flüssigkeit durch die Mündungsöffnung(en) der Pipettenspitze(n) in dieselbe(n) einzusaugen bzw. aus dieser/diesen auszustoßen. Grundsätzlich kann das Ansaugen und Ausstoßen von Flüssigkeit in bzw. aus der jeweiligen Pipettenspitze jedoch auch auf sonstige Weise erfolgen.

Der Pipettierautomat weist vorzugsweise Aufnahmeeinrichtungen für zu pipettierende Flüssigkeiten auf oder ist zur Aufnahme oder Halterung solcher Aufnahmeeinrichtungen, insbesondere sog. Näpfchen, Wells o. dgl., ausgebildet. Eine Aufnahmeeinrichtung im Sinne der vorliegenden Erfindung ist vorzugsweise ein Gefäß, eine Ausnehmung, Einbuchtung, Vertiefung und/oder vordefinierte Position, wobei die Aufnahmeeinrichtung dazu ausgebildet ist, zu pipettierende Flüssigkeit aufzunehmen und bereitzustellen.

Der Pipettierautomat weist vorzugsweise einen Aktuator oder mehrere Aktuatoren auf, mittels dem/derer die Pipettiervorrichtung zwischen unterschiedlichen Aufnahmeeinrichtungen bewegt wird oder bewegbar ist, und/oder mittels dem/derer eine Flüssigkeitsaufnahme in und/oder eine Flüssigkeitsabgabe aus einer oder mehreren der Pipettenspitzen der Pipettiervorrichtung bewirkt werden kann. Diese oder weitere Aktuatoren des Pipettierautomaten sind vorzugsweise durch eine Steuerungseinrichtung steuerbar.

Ein Aktuator im Sinne der vorliegenden Erfindung ist vorzugsweise eine Einrichtung zur gesteuerten Bewirkung eines Effekts zum Betrieb des Pipettierautomaten. Insbesondere ist der Aktuator ein Motor oder sonstiger Antrieb zur Bewegung der Pipettiervorrichtung und/oder ein Antrieb zur Bewegung des Kolbens einer Zylinder-Kolben-Anordnung oder sonstigen Verdrängereinheit, um Flüssigkeit durch eine Mündungsöffnung einer Pipettenspitze in dieselbe einzusaugen bzw. aus dieser auszustoßen.

Die EP 3 021 123 A1, von der die vorliegende Erfindung ausgeht, zeigt eine computer-implementierte Steuerungseinrichtung zum Steuern eines Pipettierautomaten der in Rede stehenden Art, mit den Merkmalen des Oberbegriffs von Anspruch 1. Die nachfolgend beschriebenen Merkmale und Erläuterungen treffen somit auch auf die vorliegende Erfindung zu.

Die bekannte Steuerungseinrichtung ist wie die erfindungsgemäße Steuerungseinrichtung zum Steuern mindestens eines Aktuators zur Bewegung einer Pipettiervorrichtung zwischen Aufnahmeeinrichtungen für zu pipettierende Flüssigkeiten ausgebildet. Die Steuerungseinrichtung ist derart zum Steuern der Pipettiervorrichtung ausgebildet, dass mittels der Pipettiervorrichtung in einem Transferschritt ein bestimmtes Transfervolumen an Flüssigkeit aus mindestens einer der Aufnahmeeinrichtungen aufnehmbar und wenigstens ein Teil des Transfervolumens an Flüssigkeit in mindestens eine andere der Aufnahmeeinrichtungen abgebbar ist.

In einem Transferschritt wird also ein bestimmtes Transfervolumen an Flüssigkeit von der Pipettiervorrichtung aus einer oder mehreren der Aufnahmeeinrichtungen aufgenommen und wenigstens ein Teil des Transfervolumens an Flüssigkeit wird von der Pipettiervorrichtung in eine oder mehrere andere der Aufnahmeeinrichtungen abgegeben. Wird nach dem Abgeben einer Flüssigkeit wieder eine Flüssigkeit von der Pipettiervorrichtung aufgenommen, so ist dieses Aufnehmen von Flüssigkeit bereits Teil eines weiteren (neuen, separaten) Transferschritts. Das Bewegen der Pipettiervorrichtung von der Aufnahmeeinrichtung, aus der zuletzt Flüssigkeit abgegeben wurde, zu der Aufnahmeeinrichtung, aus der als nächstes Flüssigkeit entnommen wird, kann dem weiteren Transferschritt zugeordnet werden.

Die bekannte Steuerungseinrichtung hat eine Eingabeeinrichtung und eine Anzeigeeinrichtung. Durch die Anzeigeeinrichtung ist mindestens eine Konfigurationsoberfläche darstellbar. Eine Eingabeeinrichtung im Sinne der vorliegenden Erfindung ist vorzugsweise eine Tastatur, eine Computermaus, ein Trackball, ein Touchscreen, eine Kamera, ein Sensor oder eine sonstige Einrichtung zur Steuerung eines oder zur Dateneingabe in einen Computer o. dgl. Eine Anzeigeeinrichtung ist vorzugsweise ein Monitor, ein Display, ein Touchscreen, ein Projektor oder eine sonstige Einrichtung zur Anzeige einer graphischen Benutzeroberfläche, insbesondere einer oder mehrerer Konfigurationsoberflächen.

Die Steuerungseinrichtung kann mindestens eine Speichereinrichtung, insbesondere einen Arbeitsspeicher und/oder einen Festspeicher wie eine Festplatte, und/oder einen Prozessor aufweisen. Ferner weist die Steuerungseinrichtung vorzugsweise eine Schnittstelle auf, die dazu ausgebildet ist, den oder die Aktuatoren des Pipettierautomaten zu steuern.

Die Steuerungseinrichtung ist dazu ausgebildet, dass in der Konfigurationsoberfläche die Aufnahmeeinrichtungen durch graphisch dargestellte Wells repräsentiert werden bzw. sind. Die Aufnahmeeinrichtungen, aus denen zu pipettierende Flüssigkeit durch die Pipettiervorrichtung aufgenommen werden soll, korrespondieren mit Quell-Wells. Die Aufnahmeeinrichtungen, in die zu pipettierende Flüssigkeit durch die Pipettiervorrichtung abgegeben werden soll, korrespondieren mit Ziel-Wells.

Ein Well im Sinne der vorliegenden Erfindung ist vorzugsweise ein graphisches Mittel zur Repräsentation mindestens einer Aufnahmeeinrichtung in einer durch die Anzeigeeinrichtung darstellbaren oder dargestellten Konfigurationsoberfläche. Das jeweilige Well korrespondiert zu einer bestimmten Aufnahmeeinrichtung des Pipettierautomaten, insbesondere in seiner Form und/oder Position bzw. Lage anderen Aufnahmeeinrichtungen bzw. Wells gegenüber. Besonders bevorzugt sind die Aufnahmeeinrichtungen des zu steuernden Pipettierautomaten mittels der Wells in der Konfigurationsoberfläche schematisch dargestellt, darstellbar oder repräsentiert.

Die Steuerungseinrichtung ist dazu ausgebildet, dass zum Spezifizieren mindestens eines aufzunehmenden und mindestens eines abzugebenden Transfervolumens für mindestens einen Transferschritt in der Konfigurationsoberfläche ein zu dem aufzunehmenden und/oder abzugebenden Transfervolumen korrespondierender Transferparameter mittels der Eingabeeinrichtung festlegbar ist.

Das Transfervolumen ist vorzugsweise ein bestimmtes Flüssigkeitsvolumen, das aus der jeweiligen Aufnahmeeinrichtung aufgenommen werden soll oder an die jeweilige Aufnahmeeinrichtung abgegeben werden soll. Das Transfervolumen kann ein aufzunehmendes bzw. zu entnehmendes Flüssigkeitsvolumen sein, also ein aus einer Aufnahmeeinrichtung zu entnehmendes und/oder in eine Pipettenspitze oder Spritze aufzunehmendes, insbesondere einzusaugendes, Volumen der zu pipettierenden Flüssigkeit. Die Begriffe "aufzunehmendes" und "zu entnehmendes" Transfervolumen sind bevorzugt synonym und austauschbar. Alternativ oder zusätzlich kann das Transfervolumen ein abzugebendes Volumen sein, also ein aus einer Pipettenspitze oder Spritze zu beförderndes Volumen und/oder in die Aufnahmeeinrichtung abzugebendes Volumen der zu pipettierenden Flüssigkeit. Aufzunehmende und abzugebende Transfervolumina können unterschieden werden, beispielsweise durch ein Vorzeichen oder eine sonstige Kennzeichnung des Transferparameters bzw. Transfervolumens.

Mittels der Eingabeeinrichtung sind in der Konfigurationsoberfläche mehrere Quell-Wells und mehrere Ziel-Wells auswählbar. Die bekannte Steuerungseinrichtung ist dazu ausgebildet, dass zum Spezifizieren mehrerer Transferschritte und deren Reihenfolge der Ausführung durch die Pipettiervorrichtung und den Aktuator in der Konfigurationsoberfläche mehrere zuvor ausgewählte Quell-Wells mindestens einem Ziel-Well durch Auswahl dieses Ziel-Wells zuordenbar sind. Vorzugsweise wird durch das Spezifizieren von Transferschritten auch deren Reihenfolge der Ausführung durch die Pipettiervorrichtung und den Aktuator festgelegt.

Das Dokument US 2004/0142486 A1 beschäftigt sich mit einer effizienten Materialhandhabung und insbesondere mit einer Probensortierung zum Gruppieren von chemischen Verbindungen. Durch die geschickte Sortierung von chemischen Verbindungen nach vorgegebenen Sortierkriterien können die Verbindungen in Chargen gruppiert werden, was ein Hochdurchsatz-Screening und andere Anwendungen erleichtert (kombinatorische Chemie).

Das Dokument WO 2017/143182 A2 beschreibt eine automatisierte Probenanalyse von einer Vielzahl biologischer Proben. Vor einer Bestückung eines Voranalyse-Systems wird eine Probenanalyse durchgeführt. Bei dem Voranalyse-Prozess werden die notwendigen Gefäße identifiziert, Gefäße mit einer bestimmten Probe gruppiert und zu einem Analysegerät transportiert, Informationen ausgelesen sowie leere Gefäße bereitgestellt. Die Gefäß-Gruppen werden von einem Analysegerät aufgenommen, das dann die notwendigen Schritte durchführt.

Das Dokument US 2018/0340949 A1 offenbart ein computerimplementiertes Verfahren zum Betreiben eines Laborsystems, das mehrere Laborinstrumente zum Verarbeiten biologischer Proben und eine Steuereinheit umfasst, die über ein Kommunikationsnetz verbunden ist. Die Laborinstrumente sollen diverse Prozessschritte bei biologischen Proben durchführen, wobei vorgefasste Kriterien verändert werden können, wie die Zuweisung von Proben zu jedem Testauftrag.

Das Dokument EP 3 171 177 A1 offenbart ein Verfahren und ein automatisiertes Laborsystem zum Verteilen einer oder mehrerer biologischer Proben auf mehrere Vertiefungen einer Mikroplatte. Eine vorgegebene Probenverteilliste oder Aufträge an Probentests sind gemäß einer bestimmten Verteilstrategie für zu spezifizierende Transferschritte abzuändern.

Bei vielen Transferschritten darf die jeweilige Flüssigkeit in den Aufnahmeeinrichtungen nicht mit einer anderen Flüssigkeit verunreinigt werden. Wenn Flüssigkeit aus einer Aufnahmeeinrichtung entnommen und dazu in eine auswechselbare Pipettenspitze oder Spritze aufgenommen wird und diese Flüssigkeit in eine weitere Aufnahmeeinrichtung abgegeben und dazu aus der Pipettenspitze oder Spritze ausgestoßen wird, ist die Pipettenspitze oder Spritze außen und innen benetzt. Wird diese Pipettenspitze oder Spritze in einem nachfolgenden Transferschritt in eine andere Flüssigkeit getaucht, bewirkt dies bereits eine Verunreinigung dieser anderen Flüssigkeit. Um dies zu vermeiden, wird grundsätzlich vor jedem Transferschritt, in dem eine andere Flüssigkeit als im unmittelbar vorangegangenen Transferschritt zu pipettieren ist, die Pipettenspitze oder Spritze gewechselt. Dazu wird die Pipettiervorrichtung mittels des Aktuators zu einem Behälter bewegt und die in dem unmittelbar vorangegangenen Transferschritt benutzte Pipettenspitze oder Spritze wird von der Pipettiervorrichtung gelöst und in den Behälter abgeworfen. Anschließend wird die Pipettiervorrichtung mittels des Aktuators zu einem Magazin bewegt und es wird eine neue Pipettenspitze oder Spritze aus dem Magazin entnommen und an der Pipettiervorrichtung lösbar befestigt. Der Wechsel der Pipettenspitze oder Spritze kostet also Zeit, verbraucht Material und verursacht zu entsorgenden Abfall.

Der vorliegenden Erfindung liegt das Problem zugrunde, den bekannten Pipettierautomaten hinsichtlich des Materialverbrauchs, insbesondere des Verbrauchs an Pipettenspitzen oder Spritzen, und/oder der für die Ausführung der spezifizierten Transferschritte benötigten Zeit zu verbessern.

Die zuvor aufgezeigte Problemstellung ist gelöst bei einer Steuerungseinrichtung mit den Merkmalen des Oberbegriffs von Anspruch 1 durch die Merkmale des kennzeichnenden Teils von Anspruch 1, durch einen Pipettierautomaten gemäß Anspruch 8, bei einem Verfahren zur Steuerung eines Pipettierautomaten mit den Merkmalen des Oberbegriffs von Anspruch 9 durch die Merkmale des kennzeichnenden Teils von Anspruch 9, durch ein Computerprogrammprodukt gemäß Anspruch 16 und durch eine Verwendung gemäß Anspruch 17. Vorteilhafte Weiterbildungen sind Gegenstand der jeweiligen Unteransprüche.

Bei der erfindungsgemäßen Steuerungseinrichtung mit den zuvor zur EP 3 021 123 A1 beschriebenen Merkmalen ist vorgesehen, dass die Steuerungseinrichtung dazu ausgebildet ist,
- vor der Ausführung mehrerer spezifizierter Transferschritte durch die Pipettiervorrichtung und den Aktuator die spezifizierte Reihenfolge der Ausführung dieser Transferschritte und die in diesen Transferschritten zu pipettierende Flüssigkeit zu analysieren und
- nach der Analyse automatisch die Reihenfolge der Ausführung dieser Transferschritte zu verändern und/oder mehrere dieser Transferschritte zusammenzufassen.

Beim Analysieren kann z. B. vorgesehen sein, dass die Steuerungseinrichtung nach Transferschritten sucht, gemäß denen gleiche Flüssigkeiten zu pipettieren sind. Im Sinne der vorliegenden Erfindung sind Flüssigkeiten gleich, wenn ihre chemische Zusammensetzung und/oder ggfs. Konzentration übereinstimmt/en. Das zu pipettierende Volumen ist nicht entscheidend. Es ist auch möglich, dass Flüssigkeiten als gleich und/oder vermischbar vorgegeben bzw. definiert werden können.

Beim Analysieren werden Transferschritte (z. B. diejenigen, gemäß denen gleiche Flüssigkeiten zu pipettieren sind) von der Steuerungseinrichtung dahingehend untersucht, ob diese Transferschritte unabhängig von anderen Transferschritten sind bzw. die Ausführung dieser Transferschritte in einer anderen als der spezifizierten Reihenfolge erfolgen darf. Eine Abhängigkeit eines Transferschrittes kann beispielsweise durch eine in der spezifizierten Reihenfolge geplante Veränderung der zu pipettierenden Flüssigkeit selbst oder durch ein spezifiziertes Vermischen mit einer anderen Flüssigkeit entstanden sein.

Die erfindungsgemäße Steuerungseinrichtung ist dazu ausgebildet, nach der Analyse aber vor der Ausführung der spezifizierten Transferschritte - typischerweise auf Basis der Analyseergebnisse - automatisch die Reihenfolge der Ausführung dieser Transferschritte zu verändern und/oder mehrere dieser Transferschritte zusammenzufassen. Dadurch kann automatisch die Reihenfolge der Ausführung dieser Transferschritte verändert werden und/oder ein Zusammenfassen von Transferschritten vorgenommen werden, ohne Aufnahmeeinrichtungen und/oder Flüssigkeit in den Aufnahmeeinrichtungen anders anzuordnen bzw. anders zu positionieren. So kann die für die Ausführung dieser Transferschritte benötigte Zeit und/oder der Materialverbrauch, insbesondere der Verbrauch an Pipettenspitzen oder Spritzen, verringert werden.

Beim Zusammenfassen von Transferschritten werden Pipettiervorgänge (Aufnehmen oder Abgeben eines bestimmten Transfervolumens an Flüssigkeit durch die Pipettiervorrichtung), die vor dem Zusammenfassen als in mehreren (separaten) Transferschritten auszuführen spezifiziert waren, als in einem Transferschritt auszuführen spezifiziert.

### Beispielhaftes Szenario 1

Sind z. B. drei Transferschritte spezifiziert, wobei
- gemäß dem ersten Transferschritt ein bestimmtes Transfervolumen an Flüssigkeit aus einer ersten Aufnahmeeinrichtung aufgenommen und das Transfervolumen an Flüssigkeit in eine zweite Aufnahmeeinrichtung abgegeben werden soll,
- gemäß dem zweiten Transferschritt ein bestimmtes Transfervolumen an Flüssigkeit aus der ersten Aufnahmeeinrichtung aufgenommen und das Transfervolumen an Flüssigkeit in eine dritte Aufnahmeeinrichtung abgegeben werden soll und
- gemäß dem dritten Transferschritt ein bestimmtes Transfervolumen an Flüssigkeit aus der ersten Aufnahmeeinrichtung aufgenommen und das Transfervolumen an Flüssigkeit in eine vierte Aufnahmeeinrichtung abgegeben werden soll,
   so wird bei der sukzessiven Ausführung dieser Transferschritte die Pipettiervorrichtung unter anderem
- zur ersten Aufnahmeeinrichtung bewegt, von der ersten zur zweiten Aufnahmeeinrichtung bewegt,
- von der zweiten zur ersten Aufnahmeeinrichtung bewegt, von der ersten zur dritten Aufnahmeeinrichtung bewegt,
- von der dritten zur ersten Aufnahmeeinrichtung bewegt und von der ersten zur vierten Aufnahmeeinrichtung bewegt.

Durch das Zusammenfassen dieser drei Transferschritte ist statt derer ein Transferschritt spezifiziert, gemäß dem
- die Summe der bestimmten Transfervolumina an Flüssigkeit aus der ersten Aufnahmeeinrichtung aufgenommen und nacheinander jeweils ein Teil des Transfervolumens an Flüssigkeit in die zweite, dritte und vierte Aufnahmeeinrichtung abgegeben werden soll.

So wird bei der Ausführung dieses Transferschritts die Pipettiervorrichtung unter anderem zur ersten Aufnahmeeinrichtung bewegt, von der ersten zur zweiten Aufnahmeeinrichtung bewegt, von der zweiten zur dritten Aufnahmeeinrichtung bewegt und von der dritten zur vierten Aufnahmeeinrichtung bewegt.

Bei der Ausführung dieses Transferschritts wird die Pipettiervorrichtung deutlich weniger weit bewegt, was Energie und Zeit einspart. Dies gilt umso mehr, wenn die erste Aufnahmeeinrichtung von der dritten und vierten Aufnahmeeinrichtung weiter entfernt ist als die zweite Aufnahmeeinrichtung von der dritten und vierten Aufnahmeeinrichtung.

### Beispielhaftes Szenario 2

Sind z. B. drei Transferschritte spezifiziert, wobei
- gemäß dem ersten Transferschritt ein bestimmtes Transfervolumen an Flüssigkeit aus einer ersten Aufnahmeeinrichtung aufgenommen und das Transfervolumen an Flüssigkeit in eine zweite Aufnahmeeinrichtung abgegeben werden soll,
- gemäß dem zweiten Transferschritt ein bestimmtes Transfervolumen an Flüssigkeit aus einer dritten Aufnahmeeinrichtung aufgenommen und das Transfervolumen an Flüssigkeit in die zweite Aufnahmeeinrichtung abgegeben werden soll und
- gemäß dem dritten Transferschritt ein bestimmtes Transfervolumen an Flüssigkeit aus einer vierten Aufnahmeeinrichtung aufgenommen und das Transfervolumen an Flüssigkeit in die zweite Aufnahmeeinrichtung abgegeben werden soll,
   so wird bei der sukzessiven Ausführung dieser Transferschritte die Pipettiervorrichtung unter anderem
- zur ersten Aufnahmeeinrichtung bewegt, von der ersten zur zweiten Aufnahmeeinrichtung bewegt,
- von der zweiten zur dritten Aufnahmeeinrichtung bewegt, von der dritten zur zweiten Aufnahmeeinrichtung bewegt,
- von der zweiten zur vierten Aufnahmeeinrichtung bewegt und von der vierten zur zweiten Aufnahmeeinrichtung bewegt.

Durch das Zusammenfassen dieser drei Transferschritte ist statt derer ein Transferschritt spezifiziert, gemäß dem
- unmittelbar nacheinander ein bestimmtes Transfervolumen an Flüssigkeit aus der ersten, dritten und vierten Aufnahmeeinrichtung aufgenommen und das gesamte aufgenommene Transfervolumen an Flüssigkeit in genau einem Pipettiervorgang in die zweite Aufnahmeeinrichtung abgegeben werden soll.

So wird bei der Ausführung dieses zusammengefassten Transferschritts die Pipettiervorrichtung unter anderem zur ersten Aufnahmeeinrichtung bewegt, von der ersten zur dritten Aufnahmeeinrichtung bewegt, von der dritten zur vierten Aufnahmeeinrichtung bewegt und von der vierten zur zweiten Aufnahmeeinrichtung bewegt.

Bei der Ausführung dieses zusammengefassten Transferschritts wird die Pipettiervorrichtung deutlich weniger weit bewegt, was Energie und Zeit einspart. Dies gilt umso mehr, wenn die zweite Aufnahmeeinrichtung von der dritten und vierten Aufnahmeeinrichtung weiter entfernt ist als die zweite Aufnahmeeinrichtung von der dritten und vierten Aufnahmeeinrichtung.

### Weitere Ausführungsformen des Zusammenfassens von Transferschritten

Vorzugsweise ist die Steuerungseinrichtung dazu ausgebildet, Transferschritte zusammenzufassen, indem Transferschritte, gemäß denen ein bestimmtes Transfervolumen an Flüssigkeit aus derselben Aufnahmeeinrichtung als Quelle aufgenommen und in bestimmte Aufnahmeeinrichtungen als Ziele abgegeben werden soll, automatisch durch einen Transferschritt ersetzt werden, gemäß dem einmalig die Summe der bestimmten Transfervolumina an Flüssigkeit aus der Quelle aufgenommen und das jeweils bestimmte Transfervolumen an Flüssigkeit in das jeweilige Ziel abgegeben werden soll.

Alternativ oder zusätzlich kann die Steuerungseinrichtung dazu ausgebildet sein, Transferschritte zusammenzufassen, indem Transferschritte, gemäß denen ein bestimmtes Transfervolumen an Flüssigkeit aus bestimmten Aufnahmeeinrichtungen als Quellen aufgenommen und in eine weitere Aufnahmeeinrichtung als dasselbe Ziel abgegeben werden soll, automatisch durch einen Transferschritt ersetzt werden, gemäß dem unmittelbar nacheinander das jeweils bestimmte Transfervolumen an Flüssigkeit aus der jeweiligen Quelle aufgenommen und einmalig die Summe der bestimmten Transfervolumina an Flüssigkeit in das Ziel abgegeben werden soll.

### Weitere erfindungsgemäße Lehre

Bei allen Szenarien und Ausführungsformen kann beim Analysieren alternativ oder zusätzlich vorgesehen sein, dass die Steuerungseinrichtung nach Transferschritten sucht, gemäß denen Flüssigkeiten gemischt werden sollen, also eine erste Flüssigkeit aus einer ersten Aufnahmeeinrichtung in eine zweite Aufnahmeeinrichtung abgegeben werden soll, in der sich bereits eine zweite Flüssigkeit oder ein Gemisch von Flüssigkeiten befinden soll, weil die zweite Flüssigkeit oder das Gemisch von Flüssigkeiten in einem oder mehreren vorangegangenen Transferschritten in die zweite Aufnahmeeinrichtung abgegeben werden soll. Bei der Analyse sucht die Steuerungseinrichtung auch nach diesen vorangegangenen Transferschritten sowie nach Transferschritten, gemäß denen gemischte Flüssigkeiten aus der zweiten Aufnahmeeinrichtung in eine dritte Aufnahmeeinrichtung transferiert werden soll.

Auch diese Analyse kann von der Steuerungseinrichtung genutzt werden, um vor der Ausführung der spezifizierten Transferschritte auf Basis der Analyseergebnisse automatisch die Reihenfolge der Ausführung dieser Transferschritte zu verändern und/oder mehrere dieser Transferschritte zusammenzufassen. So kann die für die Ausführung dieser Transferschritte benötigte Zeit und/oder der Materialverbrauch, insbesondere der Verbrauch an Pipettenspitzen oder Spritzen, verringert werden.

Die Steuerungseinrichtung kann auch zum Bestimmen von Transferschritten mit kompatiblen Flüssigkeiten bei der Analyse der Transferschritte ausgebildet sein. Bei kompatiblen Flüssigkeiten führt ein Vermischen bzw. Zusammenführen bzw. Kontakt nicht zu einer ungewollten Verunreinigung. Kompatible Flüssigkeiten können z. B. in einem oder mehreren Transferschritten zusammengeführt werden. Kompatible Flüssigkeiten können jeweils als Gemisch vorliegen, die sich in ihrer Konzentration unterscheiden.

Vorzugsweise ist die Anzeigeeinrichtung zur optisch unterscheidbaren Darstellung von Quell- und/oder Ziel-Wells mit kompatiblen Flüssigkeiten in der Konfigurationsoberfläche ausgebildet.

Bevorzugt ist, wenn die Steuerungseinrichtung dazu ausgebildet ist, automatisch die Reihenfolge der Ausführung dieser Transferschritte so zu verändern, dass Transferschritte, gemäß denen Flüssigkeit aus derselben Aufnahmeeinrichtung aufgenommen oder in dieselbe Aufnahmeeinrichtung abgegeben werden soll, unmittelbar aufeinanderfolgen. Optional können die dann unmittelbar aufeinanderfolgen Transferschritte noch automatisch durch die Steuerungseinrichtung zusammengefasst werden. Besonders bevorzugt ist es, wenn die Steuerungseinrichtung dazu ausgebildet ist, unmittelbar anschließend an die unmittelbar aufeinanderfolgen Transferschritte bzw. die zusammengefassten Transferschritte einen Wechsel einer Spitze der Pipettiervorrichtung zu veranlassen.

Auf diese Weise kann die Anzahl von Spitzenwechseln reduziert werden. Dadurch kann die bei der Ausführung der Transferschritte benötigte Zeit und die Anzahl der verbrauchten Spitzen reduziert werden.

Es hat sich als vorteilhaft erwiesen, wenn die Steuerungseinrichtung dazu ausgebildet ist, automatisch die Reihenfolge der Ausführung dieser Transferschritte so zu verändern, dass Transferschritte, gemäß denen Flüssigkeit in dieselbe Aufnahmeeinrichtung abgegeben und die so entstandene Flüssigkeit aus dieser Aufnahmeeinrichtung aufgenommen und in eine andere Aufnahmeeinrichtung abgegeben werden soll, unmittelbar aufeinanderfolgen. Besonders vorteilhaft ist es, wenn die Steuerungseinrichtung dazu ausgebildet ist, unmittelbar anschließend an die unmittelbar aufeinanderfolgen Transferschritte einen Wechsel einer Spitze der Pipettiervorrichtung zu veranlassen.

Auch auf diese Weise kann die Anzahl von Spitzenwechseln reduziert werden, wodurch wiederum die bei der Ausführung der Transferschritte benötigte Zeit und die Anzahl der verbrauchten Spitzen reduziert werden kann.

Bevorzugt ist die Steuerungseinrichtung dazu ausgebildet, die Pipettiervorrichtung und den Aktuator so zu steuern, dass
- unmittelbar nach einem zusammengefassten Transferschritt und/oder
- nach einem Transferschritt mit einer Flüssigkeit und vor einem nachfolgenden Transferschritt mit einer anderen Flüssigkeit
ein Wechsel einer Spitze der Pipettiervorrichtung durchgeführt wird.

Vorzugsweise ist die Anzeigeeinrichtung zur Anzeige der veränderten Reihenfolge der Transferschritte und/oder eines geänderten Transfervolumens in der Konfigurationsoberfläche und/oder zur Anzeige des Ablaufs der Transferschritte in einer weiteren Konfigurationsoberfläche ausgebildet.

Vorteilhafterweise sind vor oder nach der Analyse der Transferschritte mittels der Eingabeeinrichtung in der Konfigurationsoberfläche Transferschritte von der Analyse der Steuerungseinrichtung ausschließbar oder mehrere unterschiedliche Flüssigkeiten als zur Analyse der Transferschritte zu vermischen auswählbar.

Die vorschlagsgemäße Steuerungseinrichtung bietet den Vorteil einer komfortablen, schnellen und zuverlässigen Steuerung des Pipettierautomaten.

Ein weiterer, auch unabhängig realisierbarer Aspekt der vorliegenden Erfindung betrifft einen Pipettierautomaten mit der zuvor beschriebenen Steuerungseinrichtung, wobei der Pipettierautomat mindestens einen Aktuator zur Bewegung der Pipettiervorrichtung zwischen den Aufnahmeeinrichtungen aufweist, wobei der Aktuator durch die Steuerungseinrichtung steuerbar ist.

Ein weiterer, auch unabhängig realisierbarer Aspekt der vorliegenden Erfindung betrifft die Verwendung der zuvor beschriebenen Steuerungseinrichtung zur Steuerung des erläuterten Pipettierautomaten.

Ein weiterer, auch unabhängig realisierbarer Aspekt der vorliegenden Erfindung betrifft ein Verfahren zur Steuerung eines Pipettierautomaten zur Steuerung mindestens eines Aktuators zur Bewegung einer Pipettiervorrichtung zwischen Aufnahmeeinrichtungen für zu pipettierende Flüssigkeiten, mittels einer Steuerungseinrichtung.

Die Steuerungseinrichtung ist derart zum Steuern der Pipettiervorrichtung ausgebildet, dass mittels der Pipettiervorrichtung in einem Transferschritt ein bestimmtes Transfervolumen an Flüssigkeit aus mindestens einer der Aufnahmeeinrichtungen aufnehmbar und wenigstens ein Teil des Transfervolumens an Flüssigkeit in mindestens eine andere der Aufnahmeeinrichtungen abgebbar ist.

Die Steuerungseinrichtung weist eine Eingabeeinrichtung und eine Anzeigeeinrichtung auf. Durch die Anzeigeeinrichtung wird mindestens eine Konfigurationsoberfläche dargestellt. In der Konfigurationsoberfläche werden die Aufnahmeeinrichtungen durch graphisch dargestellte Wells repräsentiert.

Die Aufnahmeeinrichtungen, aus denen zu pipettierende Flüssigkeit durch die Pipettiervorrichtung aufgenommen werden soll, korrespondieren mit Quell-Wells und die Aufnahmeeinrichtungen, in die zu pipettierende Flüssigkeit durch die Pipettiervorrichtung abgegeben werden soll, korrespondieren mit Ziel-Wells.

Mittels der Eingabeeinrichtung sind in der Konfigurationsoberfläche mehrere Quell-Wells und mehrere Ziel-Wells auswählbar. Zum Spezifizieren mehrerer Transferschritte und deren Reihenfolge der Ausführung durch die Pipettiervorrichtung und den Aktuator werden in der Konfigurationsoberfläche mehrere zuvor ausgewählte Quell-Wells mindestens einem Ziel-Well durch Auswahl dieses Ziel-Wells zugeordnet.

Erfindungsgemäß ist vorgesehen, dass, vor der Ausführung mehrerer spezifizierter Transferschritte durch die Pipettiervorrichtung und den Aktuator, die Steuerungseinrichtung die spezifizierte Reihenfolge der Ausführung dieser Transferschritte und die in diesen Transferschritten zu pipettierende Flüssigkeit analysiert. Nach der Analyse verändert die Steuerungseinrichtung automatisch die Reihenfolge der Ausführung dieser Transferschritte und/oder die Steuerungseinrichtung fasst mehrere dieser Transferschritte zusammen.

Vorzugsweise bestimmt die Steuerungseinrichtung bei der Analyse Transferschritte mit kompatiblen Flüssigkeiten. Besonders bevorzugt ist, wenn die Anzeigeeinrichtung Quell- und/oder Ziel-Wells mit kompatiblen Flüssigkeiten in der Konfigurationsoberfläche optisch unterscheidbar darstellt.

Bei einer bevorzugten Ausführungsform verändert die Steuerungseinrichtung automatisch die Reihenfolge der Ausführung dieser Transferschritte so, dass Transferschritte, gemäß denen Flüssigkeit aus derselben Aufnahmeeinrichtung aufgenommen oder in dieselbe Aufnahmeeinrichtung abgegeben werden soll, unmittelbar aufeinanderfolgen. Unmittelbar anschließend wird vorzugsweise ein Wechsel einer Spitze der Pipettiervorrichtung vorgenommen.

Bei einer weiteren bevorzugten Ausführungsform verändert die Steuerungseinrichtung automatisch die Reihenfolge der Ausführung dieser Transferschritte so, dass Transferschritte, gemäß denen Flüssigkeit in dieselbe Aufnahmeeinrichtung abgegeben und die so entstandene Flüssigkeit aus dieser Aufnahmeeinrichtung aufgenommen und in eine andere Aufnahmeeinrichtung abgegeben werden soll, unmittelbar aufeinanderfolgen. Unmittelbar anschließend wird vorzugsweise ein Wechsel einer Spitze der Pipettiervorrichtung vorgenommen.

Bei einer weiteren bevorzugten Ausführungsform fasst die Steuerungseinrichtung Transferschritte zusammen, indem Transferschritte, gemäß denen ein bestimmtes Transfervolumen an Flüssigkeit aus derselben Aufnahmeeinrichtung als Quelle aufgenommen und in bestimmte Aufnahmeeinrichtungen als Ziele abgegeben werden soll, automatisch durch einen Transferschritt ersetzt werden, gemäß dem einmalig die Summe der bestimmten Transfervolumina an Flüssigkeit aus der Quelle aufgenommen und das jeweils bestimmte Transfervolumen an Flüssigkeit in das jeweilige Ziel abgegeben werden soll.

Bei einer weiteren bevorzugten Ausführungsform fasst die Steuerungseinrichtung Transferschritte zusammen, indem Transferschritte, gemäß denen ein bestimmtes Transfervolumen an Flüssigkeit aus bestimmten Aufnahmeeinrichtungen als Quellen aufgenommen und in eine weitere Aufnahmeeinrichtung als dasselbe Ziel abgegeben werden soll, automatisch durch einen Transferschritt ersetzt werden, gemäß dem unmittelbar nacheinander das jeweils bestimmte Transfervolumen an Flüssigkeit aus der jeweiligen Quelle aufgenommen und einmalig die Summe der bestimmten Transfervolumina an Flüssigkeit in das Ziel abgegeben werden soll.

Bei einer weiteren bevorzugten Ausführungsform steuert die Steuerungseinrichtung die Pipettiervorrichtung und den Aktuator so, dass
- unmittelbar nach einem zusammengefassten Transferschritt und/oder
- nach einem Transferschritt mit einer Flüssigkeit und vor einem nachfolgenden Transferschritt mit einer anderen Flüssigkeit
ein Wechsel einer Spitze der Pipettiervorrichtung durchgeführt wird.

Es ist bevorzugt, wenn vor oder nach der Analyse der Transferschritte mittels der Eingabeeinrichtung in der Konfigurationsoberfläche Transferschritte von der Analyse der Steuerungseinrichtung ausgeschlossen oder mehrere unterschiedliche Flüssigkeiten als zur Analyse der Transferschritte zu vermischen ausgewählt werden. Vorteilhafterweise wird eine bereits benutzte und von verschiedenen Arten von Flüssigkeit kontaminierte Spitze der Pipettiervorrichtung wiederverwendet, insbesondere zur nochmaligen Benutzung für mindestens einen weiteren Transferschritt.

Vorzugsweise zeigt die Anzeigeeinrichtung die veränderte Reihenfolge der Transferschritte und/oder ein geändertes Transfervolumen in der Konfigurationsoberfläche und/oder den Ablauf der Transferschritte in einer weiteren Konfigurationsoberfläche an.

Ein weiterer, auch unabhängig realisierbarer Aspekt der vorliegenden Erfindung betrifft ein computerlesbares Speichermedium, auf dem ein Programm zur Ausführung des vorschlagsgemäßen Verfahrens gespeichert ist. Ein computerlesbares Speichermedium ist insbesondere ein Speicherstick, eine Speicherkarte, ein Flash-Speicher, eine CD, eine DVD, eine Blu-Ray oder ein sonstiges Speichermedium wie eine Festplatte eines Servers, von dem das Programm zur Ausführung des vorschlagsgemäßen Verfahrens abrufbar sein kann.

Ein weiterer, auch unabhängig realisierbarer Aspekt der vorliegenden Erfindung betrifft ein Computerprogrammprodukt mit Programmcodemitteln, die dazu ausgebildet sind, das vorschlagsgemäße Verfahren auszuführen, wenn die Programmcodemittel auf einem Computer oder durch einen Prozessor ausgeführt werden. Ein Computerprogrammprodukt ist insbesondere eine Datei oder ein Datenstrom, der zum Abruf und zum Abspeichern, insbesondere auf Online-Portalen oder über das Internet, anbietbar ist.

Die vorgenannten Aspekte und Merkmale können unabhängig voneinander, insbesondere unabhängig von sonstigen Merkmalen der unabhängigen Patentansprüche, aber auch in beliebiger Kombination realisiert werden. Weitere Vorteile, Merkmale, Eigenschaften und Aspekte der vorliegenden Erfindung ergeben sich aus den Ansprüchen und der folgenden Beschreibung anhand der Zeichnung. Es zeigt:
- Fig. 1: in schematischer Darstellung einen vorschlagsgemäßen Pipettierautomaten mit einer vorschlagsgemäßen Steuerungseinrichtung,
- Fig. 2: in schematischer Darstellung eine erste Konfigurationsoberfläche der vorschlagsgemäßen Steuerungseinrichtung,
- Fig. 3: in schematischer Darstellung eine zweite Konfigurationsoberfläche der vorschlagsgemäßen Steuerungseinrichtung,
- Fig. 4: in schematischer Darstellung eine dritte Konfigurationsoberfläche der vorschlagsgemäßen Steuerungseinrichtung,
- Fig. 5: in schematischer Darstellung eine vierte Konfigurationsoberfläche der vorschlagsgemäßen Steuerungseinrichtung in einem ersten Zustand,
- Fig. 6: in schematischer Darstellung die vierte Konfigurationsoberfläche aus Fig. 5 in einem zweiten Zustand,
- Fig. 7: in schematischer Darstellung die vierte Konfigurationsoberfläche aus Fig. 5 in einem dritten Zustand,
- Fig. 8: in schematischer Darstellung die vierte Konfigurationsoberfläche aus Fig. 5 in einem vierten Zustand,
- Fig. 9: in schematischer Darstellung die vierte Konfigurationsoberfläche aus Fig. 5 in einem fünften Zustand,
- Fig. 10: in schematischer Darstellung eine fünfte Konfigurationsoberfläche der vorschlagsgemäßen Steuerungseinrichtung in einem ersten Zustand,
- Fig. 11: in schematischer Darstellung die fünfte Konfigurationsoberfläche aus Fig. 10 in einem zweiten Zustand,
- Fig. 12: in schematischer Darstellung die fünfte Konfigurationsoberfläche aus Fig. 10 in einem dritten Zustand,
- Fig. 13: in schematischer Darstellung die fünfte Konfigurationsoberfläche aus Fig. 10 in einem vierten Zustand.

In den Figuren werden für gleiche oder ähnliche Teile oder Elemente dieselben Bezugszeichen verwendet, wobei gleiche oder ähnliche Eigenschaften erreicht werden können, auch wenn von einer wiederholten Beschreibung abgesehen wird.

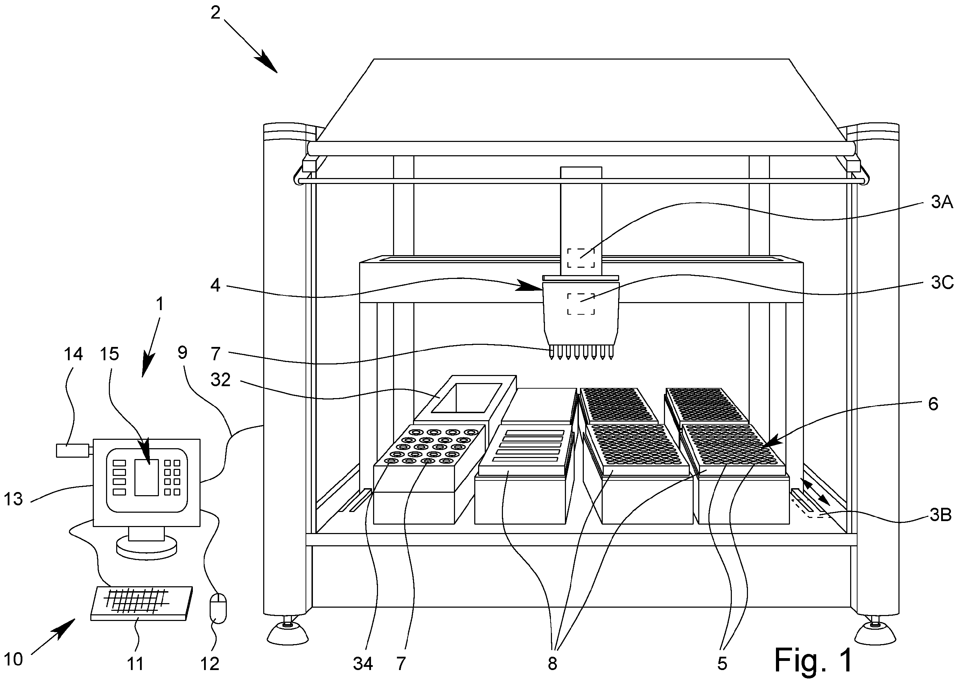

Fig. 1 zeigt in einer schematischen Darstellung eine vorschlagsgemäße Steuerungseinrichtung 1 zur Steuerung eines Pipettierautomaten 2. Die Steuerungseinrichtung 1 ist zur Steuerung mindestens eines Aktuators 3A, 3B oder 3C des Pipettierautomaten 2 ausgebildet.

Die Steuerungseinrichtung 1 ist dazu ausgebildet, einen oder mehrere der Aktuatoren 3A, 3B, 3C einer Pipettiervorrichtung 4 zwischen Aufnahmeeinrichtungen 5 für zu pipettierende Flüssigkeiten 6 zu bewegen. Alternativ oder zusätzlich ist vorgesehen, dass die Steuerungseinrichtung 1 ein oder mehrere der Aktuatoren 3A, 3B, 3C derart steuern kann, dass zu pipettierende Flüssigkeiten 6 mit der Pipettiervorrichtung 4 aufgenommen oder abgegeben werden können.

Im Darstellungsbeispiel bilden die Aktuatoren 3A, 3B eine Positioniereinrichtung zur Änderung der Position der Pipettiervorrichtung 4, bevorzugt in alle drei Raumrichtungen. Der Aktuator 3C ist vorzugsweise als Antrieb für eine Zylinder-Kolben-Einheit zum Ansaugen und Abgeben von Flüssigkeit ausgebildet. Hier gibt es jedoch auch alternative Varianten.

Die Pipettiervorrichtung 4 ist im Darstellungsbeispiel eine Mehrkanal-Pipettiervorrichtung, die vorzugsweise mehrere Pipettenspitzen 7 aufweist bzw. dazu ausgebildet ist, aus mehreren, benachbarten Aufnahmeeinrichtungen 5 Flüssigkeit 6 zu entnehmen bzw. in benachbarte Aufnahmeeinrichtungen 5 abzugeben. Die Pipettiervorrichtung 4 kann jedoch auch eine Einkanal-Pipettiervorrichtung 4 sein, bei der lediglich eine Pipettenspitze 7 vorgesehen ist.

Der Pipettierautomat 2 kann dazu ausgebildet sein, dass die Pipettiervorrichtung 4 auswechselbar ist, insbesondere zum Wechsel zwischen einer Einkanal-Pipettiervorrichtung 4 und einer Mehrkanal-Pipettiervorrichtung 4.

Die Pipettiervorrichtung 4 weist vorzugsweise den Aktuator 3C auf und ist hierdurch oder auf sonstige Weise dazu ausgebildet, Flüssigkeit 6 in die Pipettenspitze(n) 7 aufzunehmen oder hieraus abzugeben. Dies erfolgt insbesondere durch die bereits erwähnte Zylinder-Kolben-Einheit oder einen sonstigen Verdrängungsmechanismus, der durch den Aktuator 3C antreibbar sein kann.

Die Steuerungseinrichtung 1 ist derart zum Steuern der Pipettiervorrichtung 4 ausgebildet, dass mittels der Pipettiervorrichtung 4 in einem Transferschritt ein bestimmtes Transfervolumen an Flüssigkeit 6 aus mindestens einer der Aufnahmeeinrichtungen 5 in die Pipettenspitze(n) 7 aufnehmbar und wenigstens ein Teil des Transfervolumens an Flüssigkeit 6 aus der/den Pipettenspitze/n 7 in mindestens eine andere der Aufnahmeeinrichtungen 5 abgebbar ist.

Der Pipettierautomat 2 weist vorzugsweise eine oder mehrere Pipettiereinheiten 8 auf, die jeweils mehrere Aufnahmeeinrichtungen 5 aufweisen. Die Pipettiereinheiten 8 sind vorzugsweise an vorgegebenen Positionen in dem Pipettierautomaten 2 anordenbar. Vorzugsweise sind hierzu Aufnahmen, Halter oder Positionsmarkierungen vorgesehen. Ferner können die Pipettiereinheiten 8 optional mit, insbesondere unterschiedlichen, Abstandshaltern versehen sein oder werden, um einen Abstand der Aufnahmeeinrichtungen 5 zur Pipettiervorrichtung 4 bzw. der oder den Pipettenspitzen 7 einzustellen.

Bei den Pipettiereinheiten 8 handelt es sich insbesondere um sog. Mikrotiterplatten, PCR-Platten, Deep-Well-Platten und/oder Slides. Die Aufnahmeeinrichtungen 5 sind besonders bevorzugt als Gefäße, Behältnisse, Kavitäten, Ausnehmungen o. dgl., insbesondere zur Aufnahme von Flüssigkeiten oder mit einem Volumen im Mikroliterbereich ausgebildet. Alternativ oder zusätzlich können die Aufnahmeeinrichtungen 5 jedoch auch Oberflächenabschnitte zur Ablage eines Tropfens sein, die vorzugsweise entsprechend gekennzeichnet sind oder eine Oberflächenbeschichtung bzw. -strukturierung aufweisen, die von den die jeweilige Aufnahmeeinrichtung 5 umgebenden Bereichen unterschiedlich ist.

Die Steuerungseinrichtung 1 ist mit dem Pipettierautomaten 2 vorzugsweise über eine Datenschnittstelle 9 verbunden. Auf diese Weise kann die Steuerungseinrichtung 1 mit dem Pipettierautomaten 2 kommunizieren, Sensordaten aus dem Pipettierautomaten 2 abrufen oder von dem Pipettierautomaten 2 empfangen und/oder Steuersignale, insbesondere zur Steuerung eines oder mehrerer der Aktuatoren 3A, 3B, 3C, an den Pipettierautomaten 2 übermitteln.

Die Steuerungseinrichtung 1 weist vorzugsweise eine oder mehrere Eingabeeinrichtungen 10, insbesondere eine Tastatur 11 und/oder Computermaus 12, auf. Ferner weist die Steuerungseinrichtung 1 vorzugsweise eine Anzeigeeinrichtung 13, insbesondere ein Display oder Touch-Display, auf. Der Sensor eines Touch-Displays kann alternativ oder zusätzlich zu der Tastatur 11 und der Computermaus 12 als Eingabeeinrichtung 10 fungieren.

Die Steuerungseinrichtung 1 weist vorzugsweise ein computerlesbares Speichermedium 14 auf, auf dem ein Programm zur Steuerung des Pipettierautomaten 2 gespeichert sein kann. Die Steuerungseinrichtung 1 kann jedoch auch, insbesondere über das Internet, mit einem Server verbunden sein, wobei ein Computerprogrammprodukt mit Programmcodemitteln, die dazu ausgebildet sind, den Pipettierautomaten 2 zu steuern, abrufbar oder in der Ferne ausführbar ist, insbesondere als sog. Client-Server-Anwendung. Ferner weist die Steuerungseinrichtung 1 vorzugsweise einen Prozessor oder Controller auf, um ein Programm zur Steuerung des Pipettierautomaten 2 auszuführen.

Die Steuerungseinrichtung 1 ist vorzugsweise dazu ausgebildet, eine oder mehrere Konfigurationsoberflächen 15 zu erzeugen und/oder mittels der Anzeigeeinrichtung 13 darzustellen. Die Konfigurationsoberflächen 15 sind vorzugsweise dazu ausgebildet und eingerichtet, eine Steuerung des Pipettierautomaten 2 mittels der Steuerungseinrichtung 1 zu konfigurieren oder konfigurierbar zu machen. Die Steuerungseinrichtung 1 ist besonders bevorzugt dazu ausgebildet, eine oder mehrere unterschiedliche Konfigurationsoberflächen 15 zu erzeugen, worauf in den folgenden Figuren detailliert eingegangen wird.

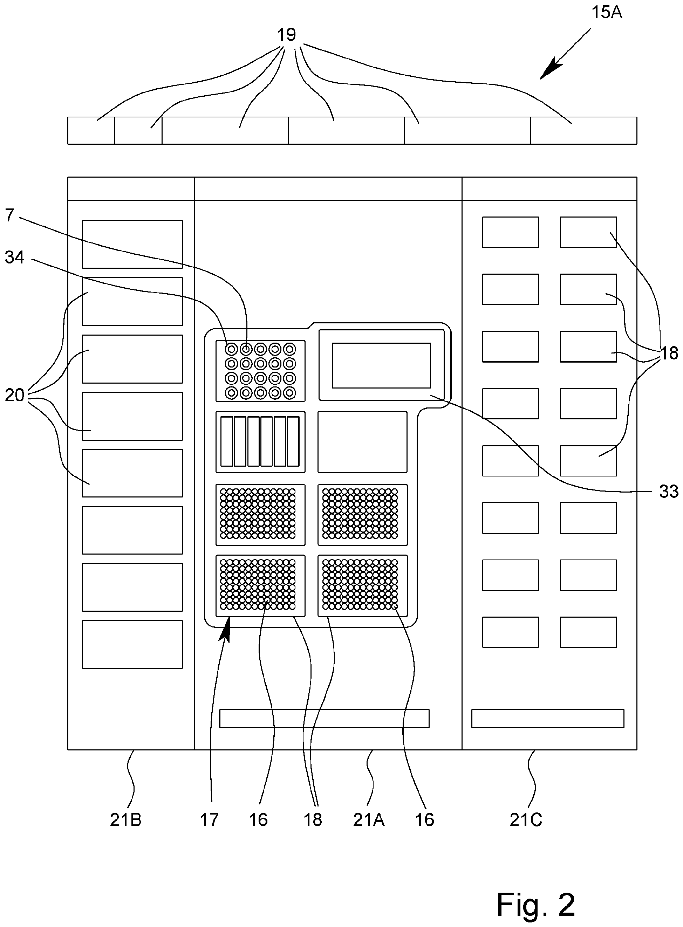

Fig. 2 zeigt in einer schematischen Darstellung eine erste Konfigurationsoberfläche 15A der vorschlagsgemäßen Steuerungseinrichtung 1. In der Konfigurationsoberfläche 15A sind mehrere Wells 16 graphisch dargestellt.

Die Wells 16 repräsentieren und korrespondieren jeweils zu Aufnahmeeinrichtungen 5, die in dem Pipettierautomaten 2 angeordnet oder anordenbar sind. Insbesondere ist vorgesehen, dass die Wells 16 die Anordnung der Aufnahmeeinrichtungen 5 im Pipettierautomaten 2 wiederspiegeln oder repräsentieren. Hierzu kann vorgesehen sein, dass die Wells 16 jeweils schematische Darstellungen der Aufnahmeeinrichtungen 5 sind oder auf sonstige Weise dazu geeignet sind, eine Aufnahmeeinrichtung 5 und, vorzugsweise, die Position der Aufnahmeeinrichtung 5 zu den sonstigen Aufnahmeeinrichtungen 5 zu repräsentieren oder zu kennzeichnen.

In der Konfigurationsoberfläche 15A ist vorzugsweise vorgesehen, dass durch Steuerung mit der Eingabeeinrichtung 10 für unterschiedliche virtuelle Positionen 17 Wells 16 auswählbar sind, wobei die Wells 16 in ihrer Position bzw. Lage und/oder Eigenschaft zu den Aufnahmeeinrichtungen 5 korrespondieren, die in dem Pipettierautomaten 2 vorgesehen sind.

Besonders bevorzugt sind korrespondierend zu Pipettiereinheiten 8 mehrere Wells 16 jeweils zu graphischen Pipettiereinheit-Äquivalenten 18 zusammengefasst, insbesondere in Form einer graphischen Repräsentation einer Platte, Mikrotiterplatte, PCR-Platte, Deep-Well-Platte und/oder einer Platte mit Slides. Die Pipettiereinheit-Äquivalente 18 korrespondieren vorzugsweise zu Pipettiereinheiten 8, die in dem Pipettierautomaten 2 angeordnet oder anordenbar sind. Vorzugsweise sind Pipettiereinheit-Äquivalente 18 in der ersten Konfigurationsoberfläche 15A auswählbar und/oder an einer oder mehreren der virtuellen Positionen 17 anordenbar.

Es kann vorgesehen sein, dass durch Positionierung von Aufnahmeeinrichtungen 5 bzw. Pipettiereinheiten 8 in dem Pipettierautomaten 2 entsprechende Wells 16 bzw. Pipettiereinheit-Äquivalente 18 automatisch an den entsprechenden virtuellen Positionen 17 vorgesehen oder angeordnet werden. Hierzu kann der Pipettierautomat 2 eine oder mehrere Sensoren aufweisen und über die Datenschnittstelle 9 entsprechende Informationen übermitteln oder zum Abruf bereitstellen, so dass die Steuerungseinrichtung 1 die Konfigurationsoberfläche 15A vollautomatisch anpasst. Die Positionierung von Pipettiereinheit-Äquivalenten 18 bzw. Wells 16 an virtuelle Positionen 17 der Konfigurationsoberfläche 15A kann jedoch auch manuell oder auf sonstige Weise erfolgen.

In Fig. 2 sind auch ein Abfallbehälter 32 für Pipettenspitzen 7 durch ein Abfallbehälter-Äquivalent 33, Pipettenspitzenhalter 34 durch Pipettenspitzenhalter-Äquivalente 35 und zum Anbringen an der Pipettiervorrichtung 4 vorgesehene Pipettenspitzen 7 durch Pipettenspitzen-Äquivalente 36 graphisch dargestellt.

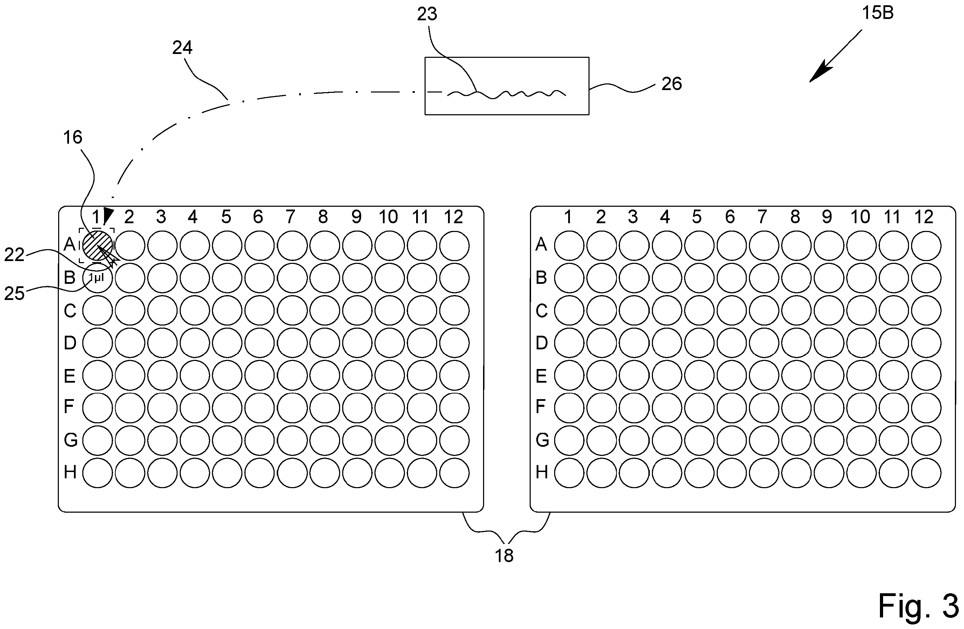

Die Konfigurationsoberfläche 15A ist vorzugsweise mit Umschaltmitteln 19 dazu ausgebildet, zwischen unterschiedlichen Funktionen umschaltbar zu sein. Insbesondere ist vorgesehen, dass die Steuerungseinrichtung 1 als Antwort auf eine Eingabe mit der Eingabeeinrichtung 10 durch Aktivierung eines der Umschaltmittel 19 die Konfigurationsoberfläche 15A derart ändert oder anpasst, dass die an den virtuellen Positionen 17 angeordneten Wells 16 und/oder Pipettiereinheit-Äquivalente 18 anwählbar oder auswählbar sind, um im Folgenden eine Konfiguration im Detail zu ermöglichen, auch Konfigurations- oder Einrichtungsmodus genannt. Ein solcher Konfigurations- oder Einrichtungsmodus ist zum Beispiel in Fig. 3 dargestellt.

Alternativ oder zusätzlich kann die Konfigurationsoberfläche 15A in einen Programmiermodus, einen Simulationsmodus und/oder einen Ausführungsmodus umgeschaltet werden, wobei im Programmiermodus grundsätzliche Funktionen oder verwendete Pipettiervorrichtungen 4 einstellbar sein können, im Simulationsmodus die Konfigurationsoberfläche 15A dazu ausgebildet ist, den Ablauf und/oder das Ergebnis einer Steuerung des Pipettierautomaten 2 darzustellen und/oder wobei im Ausführungs-Modus der Pipettierautomat 2 mit der Steuerungseinrichtung 1 in zuvor konfigurierter Weise gesteuert wird.

Die Konfigurationsoberfläche 15A ist vorzugsweise weiter dazu ausgebildet, über Konfigurationsanzeigen 20 vorgesehene Belegungen der virtuellen Positionen 17 und optional Zusatzinformationen hierzu anzuzeigen. Hierdurch ist in vorteilhafter Weise eine übersichtliche Konfiguration und Kontrolle möglich.

Die erste Konfigurationsoberfläche 15A weist vorzugsweise mehrere Anzeigeabschnitte 21A, 21B und 21C auf. In einem ersten Anzeigeabschnitt 21A sind vorzugsweise die Pipettiereinheit-Äquivalente 18 und/oder die Wells 16 vorgesehen. Der erste Anzeigeabschnitt 21A ist vorzugsweise mittig vorgesehen und/oder wird durch Betätigung der Umschaltmittel 19, die am oberen Rand vorgesehen sein können, nicht verändert.

Ein zweiter, insbesondere auf einer Seite des Anzeigeabschnitts 21A angeordneter, Anzeigeabschnitt 21B kann die Konfigurationsanzeigen 20 aufweisen. Hierbei handelt es sich um schematische Darstellungen des Anzeigeabschnitts 21A mit hervorgehobenen virtuellen Positionen 17 und/oder Beschreibungen der an der jeweils hervorgehobenen virtuellen Position 17 angeordneten Wells 16 bzw. Pipettiereinheit-Äquivalente 18.

Ferner kann in einem dritten Anzeigeabschnitt 21C, der insbesondere auf einer dem Anzeigeabschnitt 21B abgewandten Seite des Anzeigeabschnitts 21A vorgesehen sein kann, ein Konfigurationsmenü zur Einstellung unterschiedlicher Parameter, Funktionen und/oder zur Selektion einer oder mehrerer der Wells 16 bzw. Pipettiereinheit-Äquivalente 18 vorgesehen sein. Im Anzeigeabschnitt 21C sind im Einrichtungsmodus vorzugsweise unterschiedliche Pipettiereinheit-Äquivalente 18 und/oder Wells 16 auswählbar, wodurch diese den virtuellen Positionen 17 zugeordnet werden können.

Für die einzelnen Modi der Konfigurationsoberfläche 15A können alternativ oder zusätzlich auch separate Konfigurationsoberflächen 15A vorgesehen sein, vorzugsweise wobei in den jeweiligen Konfigurationsoberflächen 15A die Umschaltmittel 19 und/oder der Anzeigeabschnitt 21A jeweils zumindest im Wesentlichen identisch übernommen sind.

In einem Programmiermodus der ersten Konfigurationsoberfläche 15A, der über die Umschaltmittel 19 aktivierbar sein kann, können in dem zweiten Anzeigeabschnitt 21B bereits gewählte oder konfigurierte Verfahrensschritte oder Konfigurationen, insbesondere in einer chronologischen Reihenfolge, darstellbar sein. In dem dritten Anzeigeabschnitt 21C sind vorzugsweise Befehle, Verfahrensschritte oder Anweisungen auswählbar.

In einem optionalen Simulations-Modus der ersten Konfigurationsoberfläche 15A, der über die Umschaltmittel 19 aktivierbar sein kann, kann in der Konfigurationsoberfläche 15A und/oder mit dem Pipettierautomaten 2, insbesondere durch entsprechende Bewegungen der Pipettiervorrichtung 4, ein konfigurierter Ablauf simuliert werden.

In einem Ausführungs-Modus, der über die Umschaltmittel 19 aktivierbar sein kann, ist in dem zweiten Anzeigeabschnitt 21B oder dritten Anzeigeabschnitt 21C vorzugsweise ein Bedienfeld vorgesehen, mit dem die Steuerungseinrichtung 1 derart steuerbar ist, dass der Pipettierautomat 2 in der zuvor eingerichteten bzw. konfigurierten Weise durch die Steuerungseinrichtung 1 steuerbar ist.

Anhand der Fig. 3 bis 13 werden im Folgenden unterschiedliche weitere Konfigurationsoberflächen 15B bis 15E bzw. eine Konfigurationsoberfläche 15D bis 15E in unterschiedlichen Varianten oder Zuständen bzw. mit unterschiedlichen Bedienkonzepten näher erläutert.

Vorzugsweise ist die Steuerungseinrichtung 1 dazu ausgebildet, durch Anwahl einer oder mehrerer der Wells 16 bzw. Pipettiereinheit-Äquivalente 18 in der Konfigurationsoberfläche 15A ein Öffnen oder Generieren einer oder mehrerer der Konfigurationsoberflächen 15B bis 15E zu ermöglichen. Insbesondere wird/werden eine oder mehrere der Wells 16 bzw. Pipettiereinheit-Äquivalente 18 in dem Anzeigeabschnitt 21A ausgewählt oder markiert und durch Bedienung, insbesondere Betätigung eines Soft-Schalters, der Konfigurationsoberfläche 15A wird/werden daraufhin eine oder mehrere der Konfigurationsoberflächen 15B bis 15E generiert oder angezeigt. Hier sind jedoch auch andere Lösungen möglich.

Die Varianten bzw. Konfigurationsoberflächen 15B bis 15E und die hiermit verbundene Bedienung oder Bedienbarkeit der Steuerungseinrichtung 1 bzw. des

Pipettierautomaten 2 können auch separate, miteinander kombinierbare, auch unabhängig voneinander realisierbare Erfindungsaspekte darstellen oder bilden.

In Zusammenhang mit den Fig. 3 bis 13 werden im Folgenden Konfigurationsoberflächen 15B bis 15E vorgestellt, die zur Konfiguration bzw. Spezifizierung eines oder mehrerer Transferschritte ausgebildet sind oder anhand derer Transferschritte beschrieben werden, insbesondere durch eine chronologischen Abfolge der Schritte, die weiter unten in Zusammenhang mit der mit 1. und der mit 2. bezeichneten Seite beschrieben werden. Diese Schritte, Vorgehensweisen und dergleichen sind alternativ oder zusätzlich jedoch auch einzeln realisierbar und miteinander kombinierbar.

Fig. 3 zeigt in schematischer Darstellung eine zweite Konfigurationsoberfläche 15B der vorschlagsgemäßen Steuerungseinrichtung 1, in der exemplarisch zwei Pipettiereinheit-Äquivalente 18 mit einer Vielzahl von Wells 16 vorgesehen sind. Die Wells 16 sind innerhalb der Pipettiereinheit-Äquivalente 18 zumindest im Wesentlichen rasterartig oder auf sonstige Weise systematisch angeordnet. Vorzugsweise sind die Wells 16 jeweils in Zeilen und Spalten rasterartig angeordnet, wobei die Zeilen bzw. Spalten jeweils derart gekennzeichnet sind, dass jedes einzelne der Wells 16 durch Angabe einer Zeile und Spalte koordinatenartig adressierbar oder identifizierbar ist.

In der Konfigurationsoberfläche 15B aus Fig. 3 sind zwei unterschiedliche oder gleiche Pipettiereinheit-Äquivalente 18 nebeneinander angeordnet. Hiervon abweichend ist es jedoch auch möglich, dass lediglich ein Pipettiereinheit-Äquivalent 18, mehr als zwei Pipettiereinheit-Äquivalente 18 oder ein oder mehrere Wells 16 auch unabhängig von Pipettiereinheit-Äquivalenten 18 vorgesehen sind, vorzugsweise korrespondierend zur Belegung einer oder mehrerer virtueller Positionen 17.

Es ist insbesondere möglich, dass ein bestimmtes Pipettiereinheit-Äquivalent 18 zwei-oder mehrmals in der Konfigurationsoberfläche 15B vorgesehen ist, insbesondere nebeneinander, um in übersichtlicher Weise Transferschritte zwischen Aufnahmeeinrichtungen 16 derselben Pipettiereinheit 8 zu konfigurieren.

In der Konfigurationsoberfläche 15B ist bzw. sind eine oder mehrere Wells 16 auswählbar, insbesondere mit einem Auswahlwerkzeug 22, beispielsweise einem Cursor.

Durch Auswahl des oder der Wells 16 kann diesem/diesen vorzugsweise mindestens ein Transferparameter 23 zugeordnet werden, in Fig. 3 mit dem Pfeil 24 angedeutet. Der Pfeil 24 dient lediglich der Erläuterung und ist kein Bestandteil der Konfigurationsoberfläche 15B.

Die Steuerungseinrichtung 1 kann eine Datenbank aufweisen, in der zu den unterschiedlichen Wells 16 ein zugehöriger Transferparameter 23 abgelegt oder abgespeichert werden kann, insbesondere durch die Auswahl bzw. Zuordnung. Bei der Datenbank kann es sich um eine Tabelle oder um eine sonstige Datenstruktur handeln.

Vorzugsweise ist jedem Well 16 alternativ oder zusätzlich ein Ist-Volumen-Parameter 25 zugeordnet, der ein Flüssigkeitsvolumen repräsentiert, das in der jeweiligen Aufnahmeeinrichtung 5, die zu dem jeweiligen Well 16 korrespondiert, bereits enthalten ist und/oder nach Durchführung eines oder mehrerer Transferschritts/e im Ergebnis vorhanden sein soll. Der Ist-Volumen-Parameter 25 kann in der Konfigurationsoberfläche 15B dem jeweiligen Well 16 zugeordnet, insbesondere im Zusammenhang hiermit oder innerhalb des jeweiligen Wells 16 dargestellt oder darstellbar sein.

Der Transferparameter 23 korrespondiert vorzugsweise zu einem Transfervolumen, also zu einem Volumen an Flüssigkeit 6, das aus der jeweiligen Aufnahmeeinrichtung 5 entnommen oder hierin abgegeben werden soll.

Die Steuerungseinrichtung 1 ist vorzugsweise dazu ausgebildet, zu den Transferparametern 23, insbesondere unter Berücksichtigung oder in der Reihenfolge ihrer Vergabe, korrespondierende Steuerbefehle zur Steuerung des Pipettierautomaten 2 zu erzeugen und, vorzugsweise, über die Datenschnittstelle 9 an den Pipettierautomaten 2 zu übermitteln. Hierdurch bewirkt die Steuerungseinrichtung 1 eine Steuerung des Pipettierautomaten 2 derart, dass die Aufnahme des Transfervolumens aus oder die Abgabe des Transfervolumens in die jeweilige Aufnahmeeinrichtung 5 bewirkt wird. Dies erfolgt insbesondere durch Ansteuerung eines oder mehrerer der Aktuatoren 3A, 3B, 3C des Pipettierautomaten 2.

Gemäß einem, auch unabhängig realisierbaren Aspekt der vorliegenden Erfindung ist vorgesehen, dass die Steuerungseinrichtung 1 bzw. die Konfigurationsoberfläche 15B dazu ausgebildet ist, dass in oder mit der Konfigurationsoberfläche 15B der Transferparameter 23 einstellbar oder vorgebbar ist. Insbesondere weist die Konfigurationsoberfläche 15B eine Eingabemaske 26 oder ein Menü, ein Auswahlfeld o. dgl. auf, in die der Transferparameter 23, insbesondere mittels der Tastatur 11, eingegeben, ausgewählt, geändert oder auf sonstige Weise vorgegeben werden kann.

Der Transferparameter 23 ist vorzugsweise auf derselben Konfigurationsoberfläche 15B einstellbar, auf der auch einzelne Wells 16 auswählbar sind und der Transferparameter 23 diesen zuordenbar ist. Dies hat sich als besonders vorteilhaft für eine schnelle Konfiguration und übersichtliche Bedienung des Pipettierautomaten 2 mit der Steuerungseinrichtung 1 herausgestellt.

Der Transferparameter 23 ist oder korrespondiert besonders bevorzugt zu dem Transfervolumen, also einem Flüssigkeitsvolumen an Flüssigkeit 6, die durch die Pipettiervorrichtung 4 mit der oder den Pipettenspitzen 7 aufgenommen oder hierdurch abgegeben werden soll.

Alternativ oder zusätzlich kann der Transferparameter 23 auch eine Transferstoffmenge, insbesondere in Mol, ein Transferwerkzeug, insbesondere eine Einkanal- oder Mehrkanal-Pipettiervorrichtung 4, ein Transfermuster und/oder eine Transfer- oder Zielkonzentration sein, aufweisen oder hierzu korrespondieren. Besonders bevorzugt korrespondiert der Transferparameter 23 jedoch jeweils zu einem Transfervolumen, das aus der Transferstoffmenge, der Transfer- oder Zielkonzentration bestimmbar oder berechenbar sein kann.

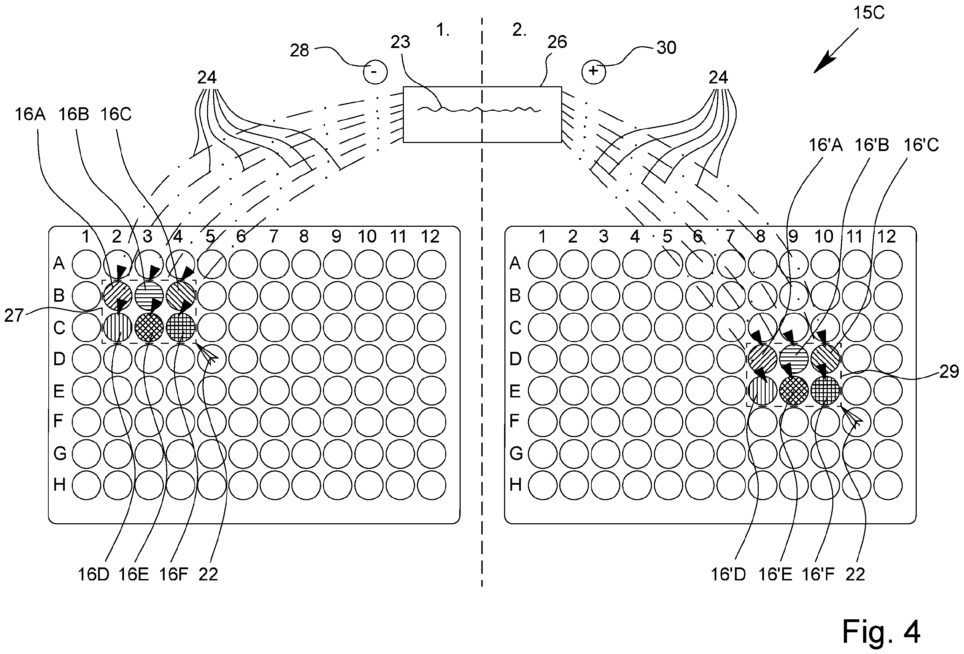

In Fig. 4 ist eine schematische Darstellung einer dritten Konfigurationsoberfläche 15C der vorschlagsgemäßen Steuerungseinrichtung 1 abgebildet.

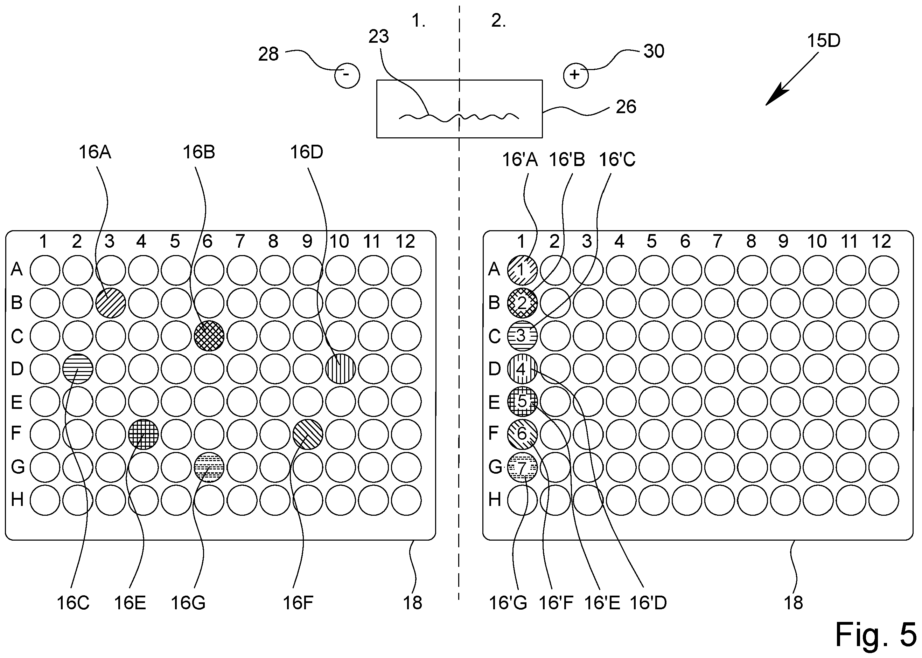

Für grundlegende Aspekte, insbesondere betreffend die Zuordnung des Transferparameters 23, wird an dieser Stelle explizit auf die Erläuterungen im Zusammenhang mit der Fig. 3 verwiesen. Im Folgenden wird daher lediglich auf weitere Besonderheiten komplexerer Steuerungsprozeduren eingegangen, vorzugsweise wobei die im Zusammenhang mit Fig. 3 beschriebenen Eigenschaften und Merkmale zugrunde liegen oder entsprechend anwendbar sind. Entsprechendes gilt für die weiteren Konfigurationsoberflächen 15D bis 15E, die in Zusammenhang mit Fig. 5 bis 13 erläutert werden.

Die Konfigurationsoberfläche 15C aus Fig. 4 ist aus Gründen des besseren Verständnisses mit einer gestrichelten Linie in einen ersten und einen zweiten Abschnitt unterteilt, wobei diese gestrichelte Linie sowie die Zahlen 1. und 2. nicht zu der Konfigurationsoberfläche 15C zählen, sondern lediglich eine bevorzugte zeitliche Abfolge darstellen sollen. In entsprechender Weise sind die Pfeile 24 ebenfalls lediglich zur Erläuterung dargestellt, werden vorzugsweise jedoch nicht in der jeweiligen Konfigurationsoberfläche 15 abgebildet oder sind Teil dieser.

In der Konfigurationsoberfläche 15C ist vorzugsweise ein Well 16 oder eine, hier mit einem gestrichelten Rand hervorgehobene, erste Gruppe 27 von Wells 16A bis 16F auf der 1. Seite ausgewählt. Durch Auswahl der Wells 16A bis 16F werden diesen Wells 16A bis 16F vorzugsweise der oder die Transferparameter 23 zugeordnet, wie mit den Pfeilen 24 angedeutet.

Der jeweilige Transferparameter 23, der in diesem ersten Schritt jeweils zugeordnet wird, ist vorzugsweise ein Transferparameter 23, der zu einem oder mehreren zu entnehmenden Transfervolumina korrespondiert, was in der Konfigurationsoberfläche 15C mit dem Minus-Symbol 28 angedeutet ist, was lediglich zu Erläuterungszwecken dient und bevorzugt nicht zu der Konfigurationsoberfläche 15C gehört oder mittels der Steuerungseinrichtung 1 anzeigbar ist.

Nach Auswahl eines ersten Wells 16A bis 16F oder mehrerer erster Wells 16A bis 16F als eine erste Gruppe 27 auf der 1. Seite kann im Folgenden eine zweite Auswahl eines zweiten Wells 16'A bis 16'F oder eine Auswahl einer zweiten Gruppe 29 zweiter Wells 16'A bis 16'F auf der 2. Seite erfolgen. Durch diese Auswahl werden vorzugsweise zu einem oder mehreren abzugebenden Transfervolumina korrespondierende Transferparameter 23 den jeweiligen zweiten Wells 16'A bis 16'F zugeordnet. In der Konfigurationsoberfläche 15C wird diese zeitliche Folge durch die mittige Strichlinie und die Bezeichnung mit 1. und 2. symbolisiert.

Es ist bevorzugt, dass bei einer auf der 1. Seite erfolgenden ersten Auswahl eines ersten Wells 16A bis 16F oder einer ersten Gruppe 27 erster Wells 16A bis 16F jedem ausgewählten ersten Well 16A bis 16F automatisch ein zu einem zu entnehmenden Transfervolumen korrespondierender Transferparameter 23 zugeordnet wird. Folgt auf diese erste Auswahl eine auf der 2. Seite erfolgende zweite Auswahl eines zweiten Wells 16'A bis 16'F oder einer Gruppe 29 zweiter Wells 16'A bis 16'F, ist es bevorzugt, wenn jedem ausgewählten zweiten Well 16'A bis 16'F automatisch ein zu einem abzugebenden Transfervolumen korrespondierender Transferparameter 23 zugeordnet wird.

Die ersten Wells 16A bis 16F, denen ein Transferparameter 23 zugeordnet wird, der zu einem zu entnehmenden Transfervolumen korrespondiert, wie auch mit dem Minus-Symbol 28 angedeutet, werden auch Quell-Wells genannt. Zweite Wells 16'A bis 16'F, denen ein Transferparameter 23 zugeordnet wird, der zu einem abzugebenden Transfervolumen korrespondiert, werden vorzugsweise auch als Ziel-Wells bezeichnet. Eine Auswahl von Wells 16 auf der 1. Seite führt also vorzugsweise automatisch zur Definition von Quell-Wells 16A bis 16F, während eine Auswahl von Wells 16 auf der 2. Seite zur Definition von Ziel-Wells 16'A bis 16'F führt.

Die Aufnahmeeinrichtungen 5, aus denen zu pipettierende Flüssigkeit 6 durch die Pipettiervorrichtung 4 aufgenommen werden soll, korrespondieren mit den Quell-Wells 16A bis 16F. Die Aufnahmeeinrichtungen 5, in die zu pipettierende Flüssigkeit 6 durch die Pipettiervorrichtung 4 abgegeben werden soll, korrespondieren mit den Ziel-Wells 16'A bis 16'F.

Mittels der Eingabeeinrichtung 10 sind in der Konfigurationsoberfläche 15C mehrere Quell-Wells 16A bis 16F und mehrere Ziel-Wells 16'A bis 16'F auswählbar. Die Steuerungseinrichtung 1 ist dazu ausgebildet, dass zum Spezifizieren mehrerer Transferschritte und deren Reihenfolge der Ausführung durch die Pipettiervorrichtung 4 und den Aktuator 3 in der Konfigurationsoberfläche 15C mehrere zuvor ausgewählte Quell-Wells 16A bis 16F mehreren Ziel-Wells 16'A bis 16'F durch Auswahl dieser Ziel-Wells 16'A bis 16'F zuordenbar sind.

Bei Auswahl einer Gruppe 27 erster Wells 16A bis 16F auf der 1. Seite und der folgenden Auswahl einer Gruppe 29 zweiter Wells 16'A bis 16'F auf der 2. Seite erfolgt vorzugsweise automatisch eine paarige 1:1 Zuordnung. Bei Nutzung einer Mehrkanal-Pipettiervorrichtung 4 korrespondierend zu den Gruppen 27, 29 wird diese 1:1 Zuordnung automatisch durch separate Pipettenspitzen 7 erreicht bzw. ermöglicht. Bei Auswahl von Gruppen 27, 29, die nicht zu einer Mehrkanal-Pipettiervorrichtung 4 korrespondieren, werden nach Auswahl der Gruppen 27, 29 Zuordnungen bzw. Transfervorgänge automatisch aufgespalten, so dass durch die Steuerung Einzeltransfers erfolgen oder einzelne, jeweils zwischen der Gruppe 27 erster Wells 16A bis 16F und der Gruppe 29 zweiter Wells 16'A bis 16'F wechselnde Auswahlen fingiert werden oder auf sonstige Weise erreicht wird, dass jeweils eine paarige Zuordnung erfolgt.

Im Darstellungsbeispiel ist die Steuerungseinrichtung 1 dazu ausgebildet und eingerichtet, in Abhängigkeit von der Anzahl der Pipettenspitzen 7 der jeweils gewählten Pipettiervorrichtung 4 den Pipettierautomaten 2 nach Auswahl zweier Gruppen 27, 29 derart zu steuern, dass in aufeinanderfolgenden Vorgängen Flüssigkeit 6 nach und nach aus den zu den ersten Wells 16A bis 16F korrespondierenden Aufnahmeeinrichtungen 5 jeweils in die zu den zweiten Wells 16'A bis 16'F korrespondierenden Aufnahmeeinrichtungen 5 transferiert wird.

In der Konfigurationsoberfläche 15C sind einander zugeordnete oder zueinander korrespondierende Wells 16 vorzugsweise zueinander zugehörig oder identisch gekennzeichnet. Im Darstellungsbeispiel weisen zueinander zugehörige oder korrespondierende Wells 16 dieselbe Schraffur auf. Alternativ oder zusätzlich können jedoch auch selbe oder gleiche Farben oder sonstige Markierungen verwendet werden.

Vorzugsweise ist ein Kontextmenü in der Konfigurationsoberfläche 15C generierbar oder aktivierbar, insbesondere durch Auswahl oder Alternativ-Auswahl der ersten Gruppe 27 bzw. der Quell-Wells 16A bis 16F. Das Kontextmenü weist vorzugsweise eine oder mehrere Optionen zur virtuellen Modifikation der Struktur bzw. Anordnung und/oder Anzahl der ausgewählten Quell-Wells 16A bis 16F der Gruppe 27 für eine folgende oder weitere Auswahl von Ziel-Wells 16'A bis 16'F auf. Die Modifikation ist insofern virtuell, als nicht die ausgewählten Quell-Wells 16A bis 16F selbst modifiziert sondern ein Transfermuster (also eine graphische Repräsentation) erzeugt und dieses modifiziert wird.

Durch Auswahl der Quell-Wells 16A bis 16F wird eine graphische Repräsentation der ausgewählten Quell-Wells 16A bis 16F dargestellt. Die graphische Repräsentation kann dann mittels der Optionen des Kontextmenüs hinsichtlich Anzahl und Anordnung der Wells verändert werden. Schließlich können anhand der graphischen Repräsentation gleichzeitig (also synchron, alle auf einmal) mehrere Ziel-Wells 16'A bis 16'F als eine zweite Gruppe 29 auf der 2. Seite ausgewählt werden. Durch die Auswahl werden die ausgewählten Quell-Wells 16A bis 16F gleichzeitig den Ziel-Wells 16'A bis 16'F zugeordnet.

Somit sind die ausgewählten Quell-Wells 16A bis 16F gleichzeitig (alle auf einmal) mehreren Ziel-Wells 16'A bis 16'F derart zuordenbar, dass sich die Anordnung und/oder Anzahl der zugeordneten Ziel-Wells 16'A bis 16'F von der Anordnung und/oder Anzahl der ausgewählten Quell-Wells 16A bis 16F unterscheidet. Dadurch werden den ausgewählten Quell-Wells 16A bis 16F und den ihnen zugeordneten Ziel-Wells 16'A bis 16'F Transferparameter 23 derart zugeordnet, dass jeweils das korrespondierende Transfervolumen aus den zu den ausgewählten Quell-Wells 16'A bis 16'C korrespondierenden Aufnahmeeinrichtungen 5 in die zu den zugeordneten Ziel-Wells 16'A bis 16'F korrespondierenden Aufnahmeeinrichtungen 5 transferierbar ist.

Wie in Fig. 4 ersichtlich sind die zugeordneten Ziel-Wells 16'A bis 16'F in derselben Konfigurationsoberfläche 15C wie die ausgewählten Quell-Wells 16A bis 16F darstellbar.

Die Steuerungseinrichtung 1 ist vorzugsweise dazu ausgebildet, den Pipettierautomaten 2 derart zu steuern, dass entweder mit einer Mehrkanal-Pipettiervorrichtung 4 oder zerlegt in mehrere Transfervorgänge mit einer Einkanal-Pipettiervorrichtung 4 ein 1:1 Volumentransfer zwischen den einzelnen Aufnahmeeinrichtungen 5 erfolgt, die einerseits zu der Gruppe 27 bzw. den Quell-Wells 16A bis 16F und andererseits zu der Gruppe 29 bzw. den Ziel-Wells 16'A bis 16'F korrespondieren. Es wird also insbesondere Flüssigkeit 6 von der Aufnahmeeinrichtung 5, die zu dem Quell-Well 16A korrespondiert, aufgenommen und nach entsprechender Bewegung der Pipettiervorrichtung 4 in die zu dem Ziel-Well16'A korrespondierende Aufnahmeeinrichtung 5 abgegeben. Entsprechendes folgt danach in Zusammenhang mit dem Quell-Well 16B und dem Ziel-Well 16'B und so weiter. Auf diese Weise ist mittels der Steuerungseinrichtung 1 und dem Pipettierautomaten 2 Flüssigkeit 6 derart transferierbar, dass Nachbarschaftsverhältnisse und Ausrichtungen von Flüssigkeiten 6 in den jeweiligen Aufnahmeeinrichtungen 5 systematisch änderbar sind.

Fig. 5 zeigt in einer schematischen Darstellung einen ersten Zustand einer vierten Konfigurationsoberfläche 15D der vorschlagsgemäßen Steuerungseinrichtung 1. In der Konfigurationsoberfläche 15D sind auf der 1. Seite mehrere ungebündelte Quell-Wells 16A bis 16G ausgewählt und dargestellt. Hierbei ist der Begriff "ungebündelt" so zu verstehen, dass sich auf einem der kürzesten (an Zeilen und Spalten orientierten) Wege zwischen mindestens zwei Wells mindestens ein nicht ausgewähltes Well befindet, also die Auswahl sich auf nicht durchgängig benachbarte Wells bezieht.

Mit dem Kontextmenü oder auch auf andere, vorzugsweise in der Konfigurationsoberfläche 15D vorgesehene Weise kann als Option vorzugsweise auswählbar sein oder ausgewählt werden, dass die ausgewählten, ungebündelten Quell-Wells 16A bis 16G gleichzeitig Ziel-Wells 16'A bis 16'G auf der 2. Seite derart zuordenbar sind, dass die Ziel-Wells 16'A bis 16'G, vorzugsweise spaltenweise, gebündelt angeordnet sind. Vorzugsweise kann dabei angegeben werden, in wie vielen nebeneinander befindlichen Spalten die Ziel-Wells 16'A bis 16'G angeordnet sein sollen.

Bei dem in Fig. 5 dargestellten Beispiel werden die auf der 1. Seite ausgewählten, ungebündelten Quell-Wells 16A bis 16G den auf der 2. Seite in Spalte 1 untereinander angeordneten Ziel-Wells 16'A bis 16'G zugeordnet. Hier wurde also als Spaltenzahl "1" gewählt.

Durch diese Zuordnung sind nun Transferschritte spezifiziert. Gemäß einem ersten Transferschritt ist Flüssigkeit 6 aus der zum Quell-Well 16A korrespondierenden Aufnahmeeinrichtung 5 (Spalte 3, Zeile B) zu entnehmen und in die zum Ziel-Well 16'A korrespondierende Aufnahmeeinrichtung 5 (Spalte 1, Zeile A) abzugeben. Gemäß einem zweiten Transferschritt ist Flüssigkeit 6 aus der zum Quell-Well 16B korrespondierenden Aufnahmeeinrichtung 5 (Spalte 6, Zeile C) zu entnehmen und in die zum Ziel-Well 16'B korrespondierende Aufnahmeeinrichtung 5 (Spalte 1, Zeile B) abzugeben. Für die übrigen Transferschritte gilt Analoges.

Die in den Ziel-Wells 16'A bis 16'G dargestellten Ziffern "1" bis "7" geben die Reihenfolge bei der Ausführung der Transferschritte durch die Pipettiervorrichtung 4 und den Aktuator 3 an. Zuerst wird also der erste spezifizierte Transferschritt ausgeführt, danach der zweite Transferschritt usw.

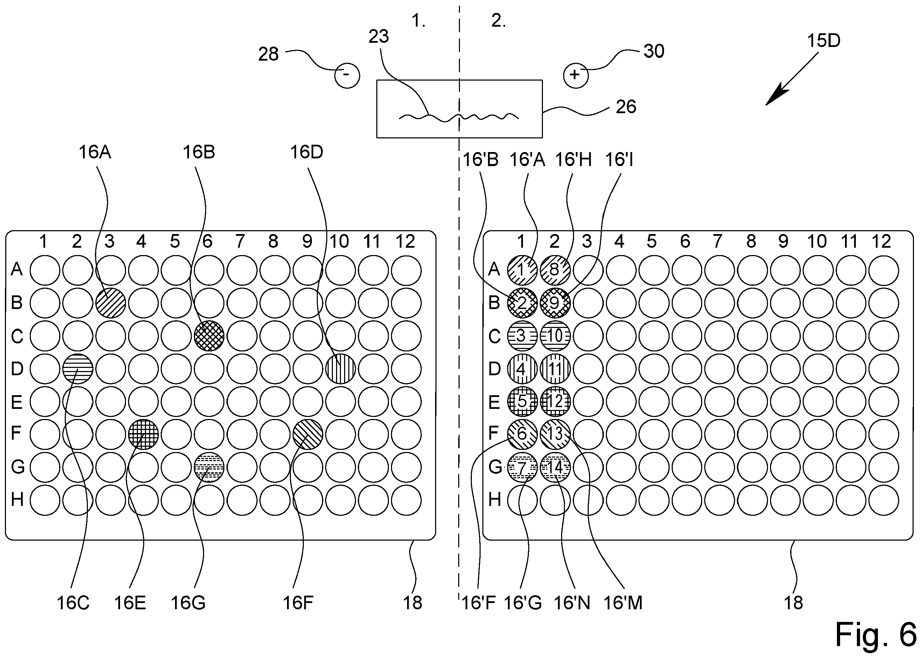

Fig. 6 zeigt in einer schematischen Darstellung einen zweiten Zustand der vierten Konfigurationsoberfläche 15D aus Fig. 5. Zusätzlich zu dem ersten bis siebten Transferschritt gemäß Fig. 5 sind beim in Fig. 6 dargestellten Zustand sieben weitere Transferschritte spezifiziert. Im Vergleich zu dem ersten bis siebten Transferschritt sind die Ziel-Wells unterschiedlich, letztere befinden sich nämlich nicht in der ersten sondern in der zweiten Spalte auf der 2. Seite. Gemäß dem achten Transferschritt ist also nochmals Flüssigkeit 6 aus der zum Quell-Well 16A korrespondierenden Aufnahmeeinrichtung 5 (Spalte 3, Zeile B) zu entnehmen aber in die zum Ziel-Well 16'H korrespondierende Aufnahmeeinrichtung 5 (Spalte 2, Zeile A) abzugeben. Gemäß dem neunten Transferschritt ist Flüssigkeit 6 aus der zum Quell-Well 16B korrespondierenden Aufnahmeeinrichtung 5 (Spalte 6, Zeile C) zu entnehmen und in die zum Ziel-Well 16`I korrespondierende Aufnahmeeinrichtung 5 (Spalte 2, Zeile B) abzugeben. Für die übrigen Transferschritte gilt Analoges.

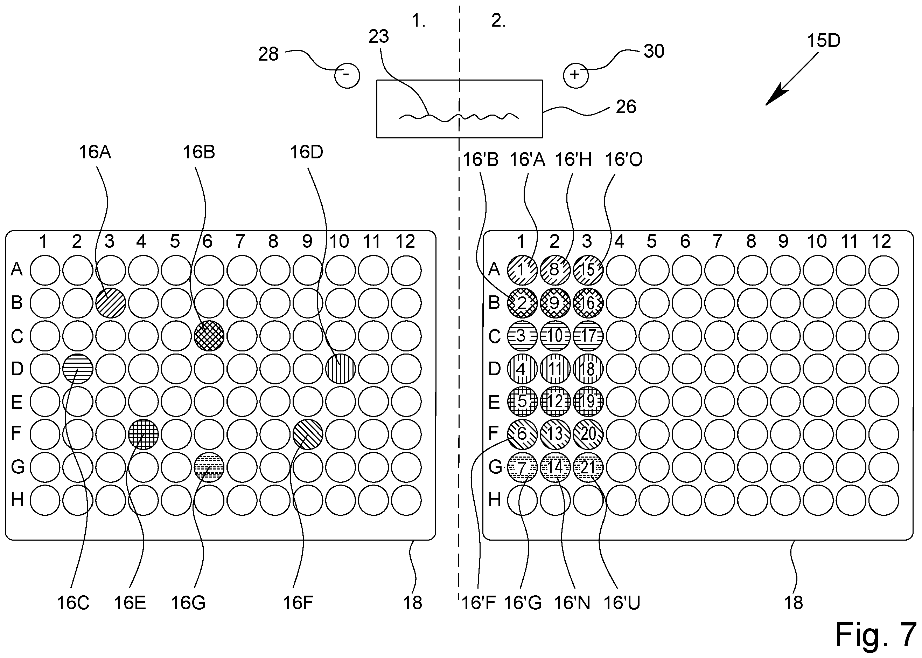

Fig. 7 zeigt in einer schematischen Darstellung einen dritten Zustand der vierten Konfigurationsoberfläche 15D aus Fig. 5. Zusätzlich zu dem ersten bis 14. Transferschritt gemäß Fig. 6 sind beim in Fig. 7 dargestellten Zustand sieben weitere Transferschritte spezifiziert. Im Vergleich zu dem ersten bis siebten Transferschritt sind die Ziel-Wells unterschiedlich, letztere befinden sich nämlich nicht in der ersten sondern in der dritten Spalte auf der 2. Seite. Gemäß dem 15. Transferschritt ist also nochmals Flüssigkeit 6 aus der zum Quell-Well 16A korrespondierenden Aufnahmeeinrichtung 5 (Spalte 3, Zeile B) zu entnehmen aber in die zum Ziel-Well 16`O korrespondierende Aufnahmeeinrichtung 5 (Spalte 3, Zeile A) abzugeben. Gemäß dem 16. Transferschritt ist Flüssigkeit 6 aus der zum Quell-Well 16B korrespondierenden Aufnahmeeinrichtung 5 (Spalte 6, Zeile C) zu entnehmen und in die zum Ziel-Well 16'P korrespondierende Aufnahmeeinrichtung 5 (Spalte 3, Zeile B) abzugeben. Für die übrigen Transferschritte gilt Analoges.

Im dargestellten und bevorzugten Ausführungsbeispiel ist die Steuerungseinrichtung 1 dazu ausgebildet, die Pipettiervorrichtung 4 und den Aktuator 3 so zu steuern, dass nach einem Transferschritt mit einer Flüssigkeit 6 und vor einem nachfolgenden Transferschritt mit einer anderen Flüssigkeit 6 ein Wechsel einer Pipettenspitze 7 der Pipettiervorrichtung 4 durchgeführt wird.

Somit würden bei dem in Fig. 7 dargestellten Beispiel nach bzw. bei jedem Transferschritt ein Wechsel von Pipettenspitzen 7 durchgeführt werden. Insgesamt wären es 21 Wechsel von Pipettenspitzen 7.

Bei der Ausführung dieser so spezifizierten Transferschritte käme es zu folgender Abfolge: Zunächst wird die Pipettiervorrichtung 4 zu einem Pipettenspitzenhalter 34 bewegt. Dort wird eine Pipettenspitze 7 an der Pipettiervorrichtung 4 befestigt. Dann wird die Pipettiervorrichtung 4 zu der Aufnahmeeinrichtung 5 verfahren, die zu dem Quell-Well 16A korrespondiert. Dort wird die Pipettiervorrichtung 4 das mittels des Transferparameters 23 definierte Transfervolumen aus dieser Aufnahmeeinrichtung 5 aufnehmen. Dann wird die Pipettiervorrichtung 4 zu der Aufnahmeeinrichtung 5 verfahren, die zu dem Ziel-Well 16'A korrespondiert. Dort wird die Pipettiervorrichtung 4 das mittels des Transferparameters 23 definierte Transfervolumen in diese Aufnahmeeinrichtung 5 abgeben. Dann wird die Pipettiervorrichtung 4 zum Abfallbehälter 32 verfahren. Dort hinein wird die benutzte Pipettenspitze 7 abgeworfen. Es folgen die weiteren Transferschritte in analoger Weise.

Zu berücksichtigen ist, dass die Wege zwischen einer Aufnahmeeinrichtung 5, die zu Quell-Wells korrespondiert, und einer Aufnahmeeinrichtung 5, die zu Ziel-Wells korrespondiert, sowie die Wege zum Abfallbehälter 32 um ein Vielfaches länger sind als die Wege zwischen zwei Aufnahmeeinrichtungen 5, die zu Quell-Wells korrespondieren, oder zwischen zwei Aufnahmeeinrichtungen 5, die zu Ziel-Wells korrespondieren.

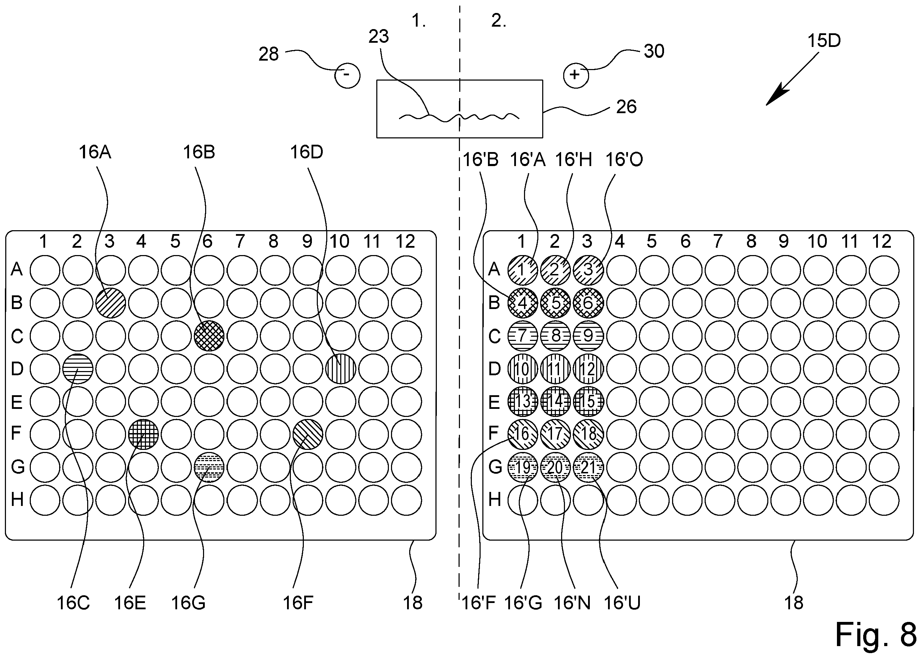

Fig. 8 zeigt in einer schematischen Darstellung einen vierten Zustand der vierten Konfigurationsoberfläche 15D aus Fig. 5. Mit dem Kontextmenü oder auch auf andere, vorzugsweise in der Konfigurationsoberfläche 15D vorgesehene Weise kann als Option vorzugsweise auswählbar sein oder ausgewählt werden, dass die Reihenfolge der Ausführung der ausgewählten Ziel-Wells 16'A bis 16'U durch die Steuerungseinrichtung 1 automatisch so verändert wird, dass Transferschritte, gemäß denen Flüssigkeit 6 aus derselben Aufnahmeeinrichtung 5 aufgenommen werden soll, unmittelbar aufeinanderfolgen.

Die Steuerungseinrichtung 1 ist nun dazu ausgebildet, vor der Ausführung der spezifizierten Transferschritte durch die Pipettiervorrichtung 4 und den Aktuator 3 die spezifizierte Reihenfolge der Ausführung dieser Transferschritte und die in diesen Transferschritten zu pipettierende Flüssigkeit 6 zu analysieren. Beim vorliegenden Ausführungsbeispiel sucht die Steuerungseinrichtung 1 beim Analysieren nach Transferschritten, gemäß denen kompatible, insbesondere gleiche Flüssigkeiten 6 zu pipettieren sind. Beim Analysieren der Transferschritte gemäß Fig. 7 wird die Steuerungseinrichtung 1 feststellen, dass gemäß den Transferschritten 1, 8 und 15 gleiche Flüssigkeiten 6 zu pipettieren sind, denn die zu pipettierenden Flüssigkeiten 6 sind alle aus der Aufnahmeeinrichtung 5 zu entnehmen, die zu dem Quell-Well 16A korrespondieren. Dies trifft auch auf die Transferschritte 2, 9, 16 und 3, 10, 17 usw. zu. Diese Transferschritte mit gleichen Flüssigkeiten 6 sind in Fig. 7 und 8 mit der gleichen Schraffur dargestellt.

Beim Analysieren werden die spezifizierten Transferschritte von der Steuerungseinrichtung 1 außerdem dahingehend untersucht, ob diese Transferschritte unabhängig von anderen Transferschritten sind bzw. die Ausführung dieser Transferschritte in einer anderen als der spezifizierten Reihenfolge erfolgen darf. Im vorliegenden Beispiel sind keine Abhängigkeiten gegeben.

Die Steuerungseinrichtung 1 ist nun so ausgebildet, dass sie nach der Analyse automatisch die Reihenfolge der Ausführung dieser analysierten Transferschritte verändert, und zwar so, dass Transferschritte, gemäß denen Flüssigkeit 6 aus derselben Aufnahmeeinrichtung 5 aufgenommen werden soll, unmittelbar aufeinanderfolgen. Die daraus resultierende geänderte Reihenfolge ist in Fig. 8 dargestellt und an der jeweiligen Ziffer in den Ziel-Wells 16'A bis 16'U erkennbar. Z. B. ist der zuvor als achter Transferschritt spezifizierte Transferschritt nun als zweiter Transferschritt auszuführen.

Bei der Ausführung dieser so spezifizierten Transferschritte käme es nun zu folgender Abfolge: Zunächst wird die Pipettiervorrichtung 4 zu einem Pipettenspitzenhalter 34 bewegt. Dort wird eine Pipettenspitze 7 an der Pipettiervorrichtung 4 befestigt. Dann wird die Pipettiervorrichtung 4 zu der Aufnahmeeinrichtung 5 verfahren, die zu dem Quell-Well 16A korrespondiert. Dort wird die Pipettiervorrichtung 4 das mittels des Transferparameters 23 definierte Transfervolumen aus dieser Aufnahmeeinrichtung 5 aufnehmen. Dann wird die Pipettiervorrichtung 4 zu der Aufnahmeeinrichtung 5 verfahren, die zu dem Ziel-Well 16'A korrespondiert. Dort wird die Pipettiervorrichtung 4 das mittels des Transferparameters 23 definierte Transfervolumen in diese Aufnahmeeinrichtung 5 abgeben. Nun erfolgt kein Wechsel der Pipettenspitze 7, sondern die Pipettiervorrichtung 4 wird zu der Aufnahmeeinrichtung 5 verfahren, die zu dem Quell-Well 16A korrespondiert. Dort wird die Pipettiervorrichtung 4 das mittels des Transferparameters 23 definierte Transfervolumen aus dieser Aufnahmeeinrichtung 5 aufnehmen. Dann wird die Pipettiervorrichtung 4 zu der Aufnahmeeinrichtung 5 verfahren, die zu dem Ziel-Well 16'H korrespondiert. Dort wird die Pipettiervorrichtung 4 das mittels des Transferparameters 23 definierte Transfervolumen in diese Aufnahmeeinrichtung 5 abgeben. Nun erfolgt wiederum kein Wechsel der Pipettenspitze 7, sondern die Pipettiervorrichtung 4 wird erneut zu der Aufnahmeeinrichtung 5 verfahren, die zu dem Quell-Well 16A korrespondiert. Dort wird die Pipettiervorrichtung 4 das mittels des Transferparameters 23 definierte Transfervolumen aus dieser Aufnahmeeinrichtung 5 aufnehmen. Dann wird die Pipettiervorrichtung 4 zu der Aufnahmeeinrichtung 5 verfahren, die zu dem Ziel-Well 16`O korrespondiert. Dort wird die Pipettiervorrichtung 4 das mittels des Transferparameters 23 definierte Transfervolumen in diese Aufnahmeeinrichtung 5 abgeben. Dann wird die Pipettiervorrichtung 4 zum Abfallbehälter 32 verfahren. Dort hinein wird die benutzte Pipettenspitze 7 abgeworfen. Es folgen die weiteren Transferschritte 4 bis 21 in analoger Weise.

Insgesamt werden hier nur sieben statt 21 Wechsel von Pipettenspitzen 7 vorgenommen, nämlich nach den Transferschritten 3, 6, 9, 12, 15, 18 und 21. Auf diese Weise wird Material (Pipettenspitzen 7) und Zeit (für die Wege zum Abfallbehälter und zurück) gespart.

Die Steuerungseinrichtung 1 ist hier auch dazu ausgebildet, automatisch die Reihenfolge der Ausführung dieser Transferschritte so zu verändern, dass Transferschritte, gemäß denen Flüssigkeit 6 in dieselbe Aufnahmeeinrichtung 5 abgegeben werden soll, unmittelbar aufeinanderfolgen. Im vorliegenden Beispiel sind solche Transferschritte jedoch nicht spezifiziert.

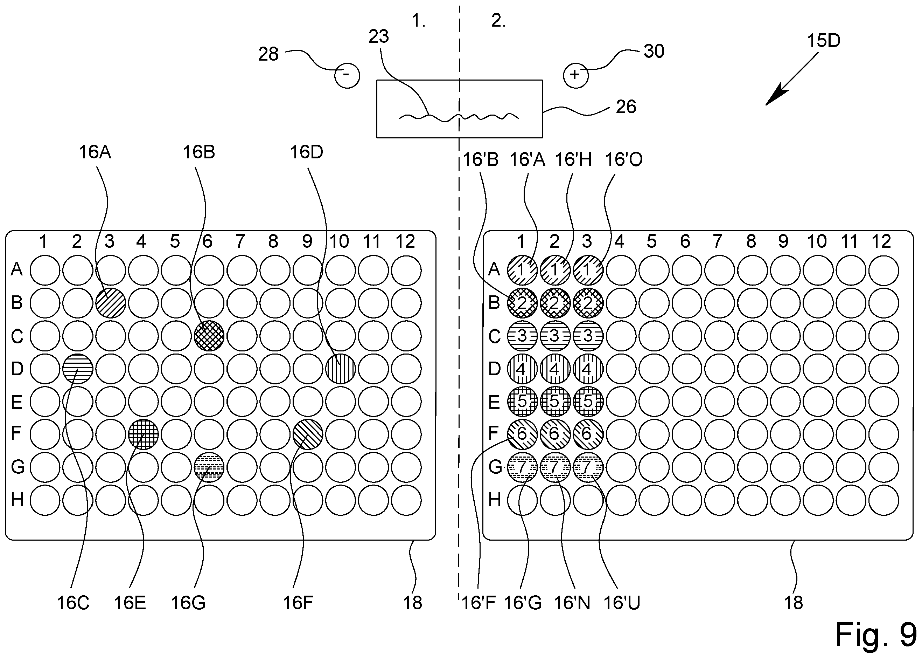

Fig. 9 zeigt in einer schematischen Darstellung einen fünften Zustand der vierten Konfigurationsoberfläche 15D aus Fig. 5. Automatisch nach der zuvor erläuterten Änderung der Reihenfolge der spezifizierten Transferschritte oder mit dem Kontextmenü oder auch auf andere, vorzugsweise in der Konfigurationsoberfläche 15D vorgesehene Weise kann vorgesehen sein oder veranlasst werden, dass Transferschritte durch die Steuerungseinrichtung 1 zusammengefasst werden, indem Transferschritte, gemäß denen ein bestimmtes Transfervolumen an Flüssigkeit 6 aus derselben Aufnahmeeinrichtung 5 als Quelle aufgenommen und in bestimmte Aufnahmeeinrichtungen 5 als Ziele abgegeben werden soll, automatisch durch einen Transferschritt ersetzt werden, gemäß dem einmalig die Summe der bestimmten Transfervolumina an Flüssigkeit 6 aus der Quelle 5 aufgenommen und das jeweils bestimmte Transfervolumen an Flüssigkeit 6 in das jeweilige Ziel 5 abgegeben werden soll.

So ist gemäß den Transferschritten 1 bis 3 aus Fig. 8 ein bestimmtes Transfervolumen an Flüssigkeit 6 aus der Aufnahmeeinrichtung 5, die zum Quell-Well 16A korrespondiert, als Quelle aufzunehmen und in die Aufnahmeeinrichtungen 5, die zu den Ziel-Wells 16'A, 16'H, 16'O korrespondieren, als Ziele abzugeben. Die Steuerungseinrichtung 1 ersetzt diese Transferschritte 1 bis 3 automatisch durch einen neuen Transferschritt 1, gemäß dem einmalig die Summe der bestimmten Transfervolumina an Flüssigkeit 6 aus der Quelle 5 aufgenommen und das jeweils bestimmte Transfervolumen an Flüssigkeit 6 in das jeweilige Ziel 5 abgegeben werden soll. Analoges gilt für die Transferschritte 4, 5, 6 und 7, 8, 9 usw.

Bei der Ausführung dieser so zusammengefassten Transferschritte käme es nun zu folgender Abfolge: Zunächst wird die Pipettiervorrichtung 4 zu einem Pipettenspitzenhalter 34 bewegt. Dort wird eine Pipettenspitze 7 an der Pipettiervorrichtung 4 befestigt. Dann wird die Pipettiervorrichtung 4 zu der Aufnahmeeinrichtung 5 verfahren, die zu dem Quell-Well 16A korrespondiert. Dort wird die Pipettiervorrichtung 4 einmalig die Summe der bestimmten Transfervolumina an Flüssigkeit 6 aus dieser Aufnahmeeinrichtung 5 aufnehmen. Dann wird die Pipettiervorrichtung 4 zu der Aufnahmeeinrichtung 5 verfahren, die zu dem Ziel-Well 16'A korrespondiert. Dort wird die Pipettiervorrichtung 4 das mittels des Transferparameters 23 definierte Transfervolumen in diese Aufnahmeeinrichtung 5 abgeben. Nun erfolgt kein Wechsel der Pipettenspitze 7, sondern die Pipettiervorrichtung 4 wird zu der Aufnahmeeinrichtung 5 verfahren, die zu dem Ziel-Well 16'H korrespondiert. Dort wird die Pipettiervorrichtung 4 das mittels des Transferparameters 23 definierte Transfervolumen in diese Aufnahmeeinrichtung 5 abgeben. Nun erfolgt wiederum kein Wechsel der Pipettenspitze 7, sondern die Pipettiervorrichtung 4 wird zu der Aufnahmeeinrichtung 5 verfahren, die zu dem Ziel-Well 16`O korrespondiert. Dort wird die Pipettiervorrichtung 4 das mittels des Transferparameters 23 definierte Transfervolumen in diese Aufnahmeeinrichtung 5 abgeben. Dann wird die Pipettiervorrichtung 4 zum Abfallbehälter 32 verfahren. Dort hinein wird die benutzte Pipettenspitze 7 abgeworfen. Es folgen die weiteren Transferschritte 2 bis 7 in analoger Weise.

Insgesamt werden auch hier nur sieben Wechsel von Pipettenspitzen 7 vorgenommen, nämlich nach den Transferschritten 1 bis 7. Außerdem wird die Pipettiervorrichtung 4 zu jeder Quelle 5 nur genau einmal verfahren. Auf diese Weise wird Material (Pipettenspitzen 7) und Zeit (für die Wege zum Abfallbehälter 32 und zurück und für die Wege zwischen Quellen 5 und Zielen 5) gespart.

Die Steuerungseinrichtung 1 ist hier auch dazu ausgebildet, automatisch Transferschritte zusammenzufassen, indem Transferschritte, gemäß denen ein bestimmtes Transfervolumen an Flüssigkeit 6 aus bestimmten Aufnahmeeinrichtungen 5 als Quellen aufgenommen und in eine weitere Aufnahmeeinrichtung 5 als dasselbe Ziel abgegeben werden soll, automatisch durch einen Transferschritt ersetzt werden, gemäß dem unmittelbar nacheinander das jeweils bestimmte Transfervolumen an Flüssigkeit 6 aus der jeweiligen Quelle aufgenommen und einmalig die Summe der bestimmten Transfervolumina an Flüssigkeit 6 in das Ziel abgegeben werden soll. Im vorliegenden Beispiel sind solche Transferschritte jedoch nicht spezifiziert.

Vorzugsweise ist vorgesehen, dass vor oder nach der Analyse der Transferschritte mittels der Eingabeeinrichtung 10 in der Konfigurationsoberfläche 15D Transferschritte von der Analyse der Steuerungseinrichtung 1 ausschließbar oder mehrere unterschiedliche Flüssigkeiten 6 als zur Analyse der Transferschritte zu vermischen auswählbar sind.

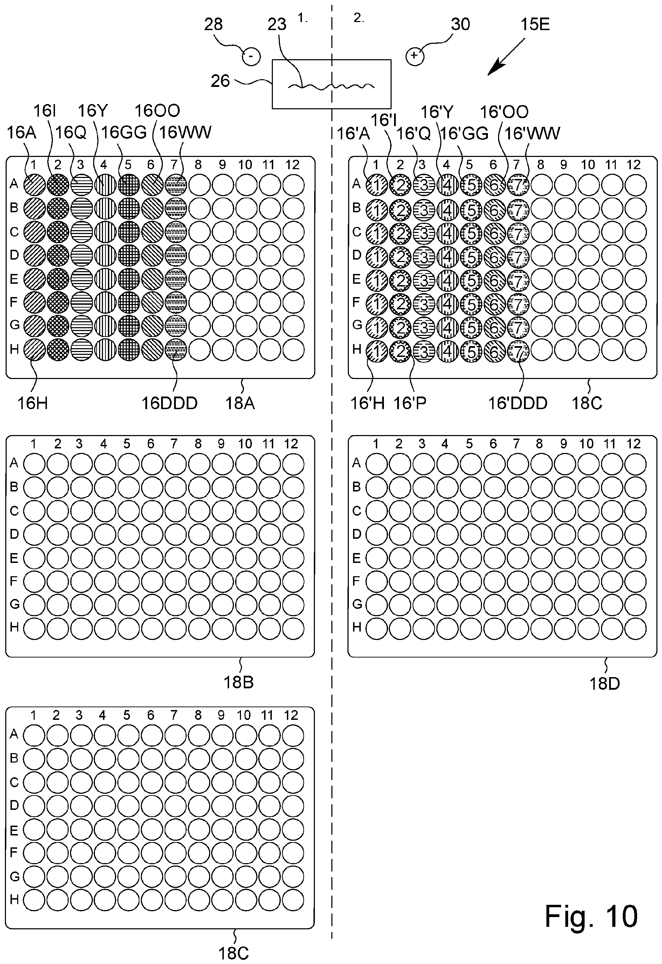

Fig. 10 zeigt in einer schematischen Darstellung einen ersten Zustand einer fünften Konfigurationsoberfläche 15E der vorschlagsgemäßen Steuerungseinrichtung 1. In der Konfigurationsoberfläche 15E ist für die Pipettiervorrichtung 4 eine Mehrkanal-Pipettiervorrichtung vorgesehen, die acht Pipettenspitzen 7 aufweist und dazu ausgebildet ist, aus acht benachbarten Aufnahmeeinrichtungen 5 Flüssigkeit 6 zu entnehmen bzw. in acht benachbarte Aufnahmeeinrichtungen 5 abzugeben.

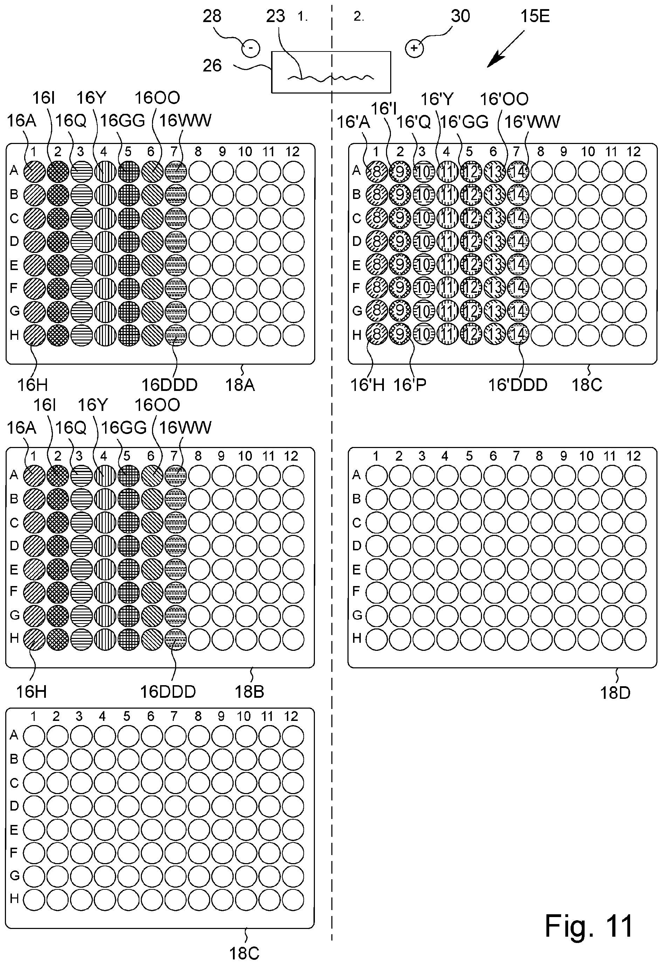

In der Konfigurationsoberfläche 15E sind auf der 1. Seite mehrere Quell-Wells 16A bis 16DDD auf dem Pipettiereinheit-Äquivalent 18A (als erste Quell-Platte) und auf der 2. Seite mehrere Ziel-Wells 16'A bis 16`DDD auf dem Pipettiereinheit-Äquivalent 18C (als erste Ziel-Platte) ausgewählt und dargestellt. Die gleichen Schraffuren in einer Spalte (1 bis 7) deuten an, dass in einem (ersten) Transferschritt mit der Mehrkanal-Pipettiervorrichtung 4 gleichzeitig aus den Aufnahmeeinrichtungen 5, die zu den Quell-Wells 16A bis 16H des Pipettiereinheit-Äquivalents 18A korrespondieren, Flüssigkeiten 6 aufgenommen werden sollen. In diesem Transferschritt sollen die Flüssigkeiten 6 in die Aufnahmeeinrichtungen 5, die zu den Ziel-Wells 16'A bis 16'H des Pipettiereinheit-Äquivalents 18C korrespondieren, abgegeben werden. In einem weiteren (zweiten) Transferschritt sollen mit der Mehrkanal-Pipettiervorrichtung 4 gleichzeitig aus den Aufnahmeeinrichtungen 5, die zu den Quell-Wells 16I bis 16P des Pipettiereinheit-Äquivalent 18A korrespondieren, Flüssigkeiten 6 aufgenommen werden. In diesem zweiten Transferschritt sollen die Flüssigkeiten 6 in die Aufnahmeeinrichtungen 5, die zu den Ziel-Wells 16'I bis 16'P des Pipettiereinheit-Äquivalents 18C korrespondieren, abgegeben werden. Für die übrigen Transferschritte gilt Entsprechendes. Die Ziffern "1" bis "7" in den Ziel-Wells 16'A bis 16'DDD geben die Reihenfolge der Ausführung der spezifizierten Transferschritte an. Fig. 11 zeigt in einer schematischen Darstellung einen zweiten Zustand der fünften Konfigurationsoberfläche 15E aus Fig. 10. In der Konfigurationsoberfläche 15E ist das Pipettiereinheit-Äquivalent 18A im Zustand der Fig. 10 dargestellt. Außerdem sind auf dem Pipettiereinheit-Äquivalent 18B (als zweite Quell-Platte) auf der 1. Seite mehrere Quell-Wells 16A bis 16DDD dargestellt. Die im Vergleich zu Fig. 10 höheren Ziffern "8" bis "14" in den Ziel-Wells 16'A bis 16'DDD des Pipettiereinheit-Äquivalents 18C geben an, dass in Bezug auf diese Ziel-Wells zusätzliche Transferschritte spezifiziert sind.

Und zwar sollen in einem achten Transferschritt mit der Mehrkanal-Pipettiervorrichtung 4 gleichzeitig aus den Aufnahmeeinrichtungen 5, die zu den Quell-Wells 16A bis 16H des Pipettiereinheit-Äquivalents 18B (als zweite Quell-Platte) korrespondieren, Flüssigkeiten 6 aufgenommen werden. In diesem Transferschritt sollen die Flüssigkeiten 6 in die Aufnahmeeinrichtungen 5, die zu den Ziel-Wells 16'A bis 16'H des Pipettiereinheit-Äquivalents 18C korrespondieren, abgegeben werden. Im neunten Transferschritt sollen mit der Mehrkanal-Pipettiervorrichtung 4 gleichzeitig aus den Aufnahmeeinrichtungen 5, die zu den Quell-Wells 16I bis 16P des Pipettiereinheit-Äquivalent 18B korrespondieren, Flüssigkeiten 6 aufgenommen werden. In diesem zweiten Transferschritt sollen die Flüssigkeiten 6 in die Aufnahmeeinrichtungen 5, die zu den Ziel-Wells 16'I bis 16'P des Pipettiereinheit-Äquivalents 18C korrespondieren, abgegeben werden. Für die übrigen Transferschritte gilt Entsprechendes.

Insgesamt betrachtet werden die Flüssigkeiten 6 der Aufnahmeeinrichtungen 5, die zu den Quell-Wells 16A bis 16H des Pipettiereinheit-Äquivalents 18A korrespondieren, mit den Flüssigkeiten 6 der Aufnahmeeinrichtungen 5, die zu den Quell-Wells 16A bis 16H des Pipettiereinheit-Äquivalents 18B gemischt, und zwar in den Aufnahmeeinrichtungen 5, die zu den Ziel-Wells 16'A bis 16'H des Pipettiereinheit-Äquivalents 18C korrespondieren.

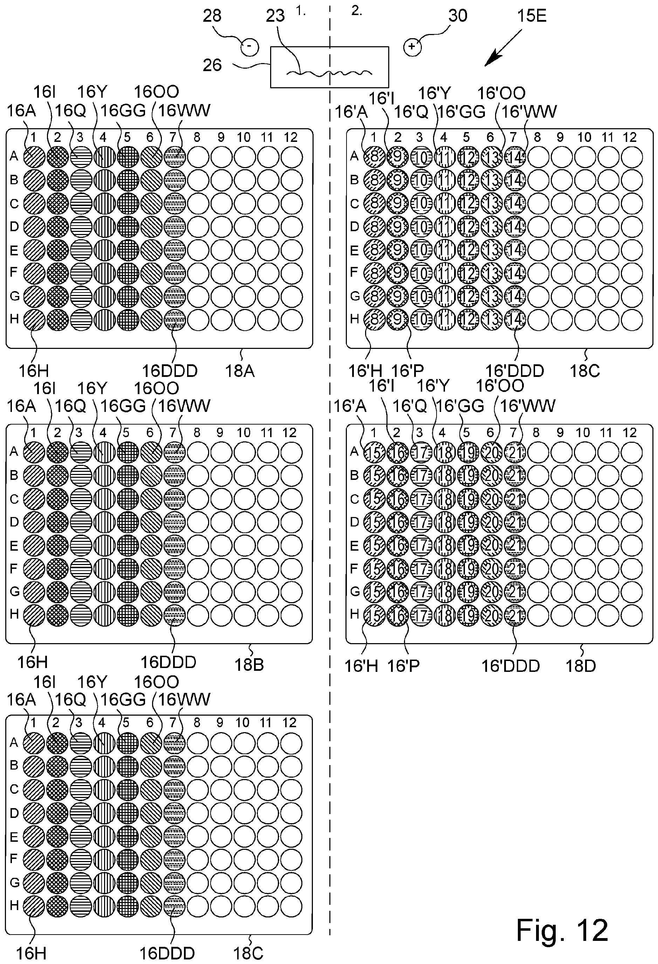

Fig. 12 zeigt in einer schematischen Darstellung einen dritten Zustand der fünften Konfigurationsoberfläche 15E aus Fig. 10. In der Konfigurationsoberfläche 15E sind die Pipettiereinheit-Äquivalente 18A und 18B im Zustand der Fig. 11 dargestellt.

Bei diesem Beispiel sollen die als Gemische vorliegenden Flüssigkeiten 6 in den Aufnahmeeinrichtungen 5, die zu den Ziel-Wells 16'A bis 16'H des Pipettiereinheit-Äquivalents 18C korrespondieren, in Aufnahmeeinrichtungen 5, die zu Ziel-Wells 16'A bis 16'H eines Pipettiereinheit-Äquivalents 18D (als zweite Ziel-Platte) korrespondieren, transferiert werden. Dabei dient die Pipettiereinheit, die zum zuvor als Ziel-Platte fungierende Pipettiereinheit-Äquivalent 18C korrespondiert, nun als Quelle (der als Gemische vorliegenden Flüssigkeiten 6) und somit das Pipettiereinheit-Äquivalent 18C nun als Quell-Platte.

Also sind auf dem Pipettiereinheit-Äquivalent 18C (nun als dritte Quell-Platte) auf der 1. Seite mehrere Quell-Wells 16A bis 16DDD ausgewählt und dargestellt. Auf der 2. Seite sind mehrere Ziel-Wells 16'A bis 16`DDD auf dem Pipettiereinheit-Äquivalent 18D (als zweite Ziel-Platte) ausgewählt und dargestellt. Die Ziffern "15" bis "21" in den Ziel-Wells 16'A bis 16'DDD des Pipettiereinheit-Äquivalents 18D geben die Reihenfolge der in diesem Zustand zusätzlich spezifizierten Transferschritte an.

In einem 15. Transferschritt sollen mit der Mehrkanal-Pipettiervorrichtung 4 gleichzeitig aus den Aufnahmeeinrichtungen 5, die zu den Quell-Wells 16A bis 16H des Pipettiereinheit-Äquivalents 18C korrespondieren, die gemischten Flüssigkeiten 6 aufgenommen werden. In diesem Transferschritt sollen die gemischten Flüssigkeiten 6 in die Aufnahmeeinrichtungen 5, die zu den Ziel-Wells 16'A bis 16'H des Pipettiereinheit-Äquivalents 18D korrespondieren, abgegeben werden. Im 16. Transferschritt sollen mit der Mehrkanal-Pipettiervorrichtung 4 gleichzeitig aus den Aufnahmeeinrichtungen 5, die zu den Quell-Wells 16I bis 16P des Pipettiereinheit-Äquivalent 18C korrespondieren, die gemischten Flüssigkeiten 6 aufgenommen werden. In diesem zweiten Transferschritt sollen die gemischten Flüssigkeiten 6 in die Aufnahmeeinrichtungen 5, die zu den Ziel-Wells 16'I bis 16'P des Pipettiereinheit-Äquivalents 18D korrespondieren, abgegeben werden. Für die übrigen Transferschritte gilt Entsprechendes.

Im dargestellten und bevorzugten Ausführungsbeispiel ist die Steuerungseinrichtung 1 dazu ausgebildet, die Mehrkanal-Pipettiervorrichtung 4 und den Aktuator 3 so zu steuern, dass nach einem Transferschritt mit einer Flüssigkeit 6 und vor einem nachfolgenden Transferschritt mit einer anderen Flüssigkeit 6 ein Wechsel aller Pipettenspitzen 7 der Mehrkanal-Pipettiervorrichtung 4 durchgeführt wird.

Somit würden bei dem in Fig. 12 dargestellten Beispiel nach jedem Transferschritt alle acht Pipettenspitzen 7 der Mehrkanal-Pipettiervorrichtung 4 ausgewechselt werden. Insgesamt wären es 21 Wechsel von je acht Pipettenspitzen 7. Es würden also 168 Pipettenspitzen weggeworfen werden. Zeitaufwendig sind die Wege zum Abfallbehälter 32, zu den Pipettenspitzenhaltern 34 und jeweils zurück.

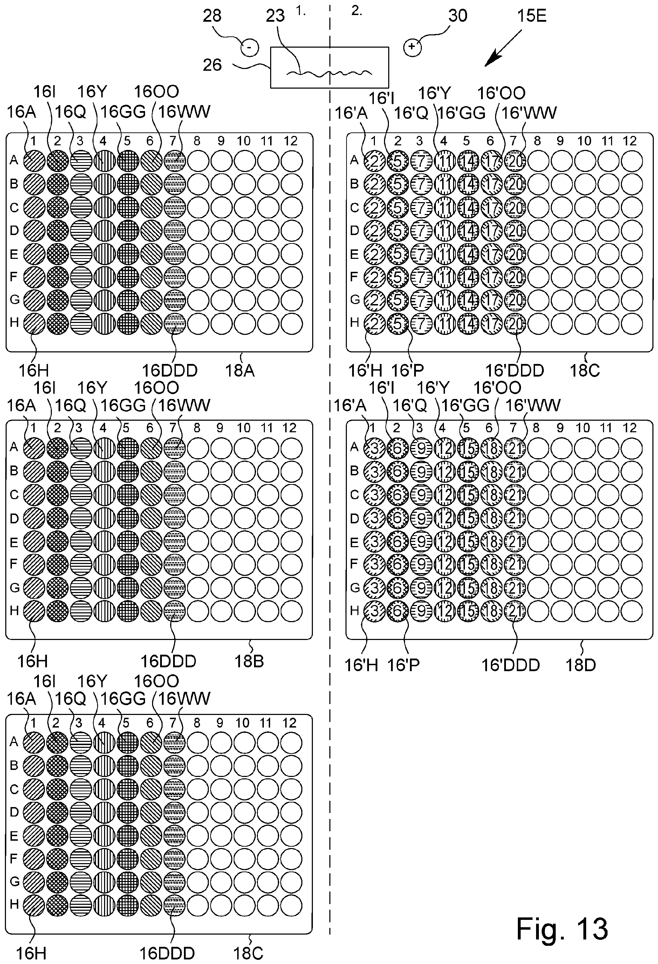

Fig. 13 zeigt in einer schematischen Darstellung einen vierten Zustand der fünften Konfigurationsoberfläche 15E aus Fig. 10. Mit dem Kontextmenü oder auch auf andere, vorzugsweise in der Konfigurationsoberfläche 15E vorgesehene Weise kann als Option vorzugsweise auswählbar sein oder ausgewählt werden, dass die Steuerungseinrichtung 1 automatisch die Reihenfolge der Ausführung dieser Transferschritte so verändert, dass Transferschritte, gemäß denen Flüssigkeit 6 in dieselbe Aufnahmeeinrichtung 5 abgegeben und die so entstandene Flüssigkeit 6 aus dieser Aufnahmeeinrichtung 5 aufgenommen und in eine andere Aufnahmeeinrichtung 5 abgegeben werden soll, unmittelbar aufeinanderfolgen.

Auch bei diesem Ausführungsbeispiel ist die Steuerungseinrichtung 1 dazu ausgebildet, vor der Ausführung der spezifizierten Transferschritte durch die Mehrkanal-Pipettiervorrichtung 4 und den Aktuator 3 die spezifizierte Reihenfolge der Ausführung dieser Transferschritte und die in diesen Transferschritten zu pipettierende Flüssigkeiten 6 zu analysieren. Beim vorliegenden Ausführungsbeispiel sucht die Steuerungseinrichtung 1 beim Analysieren nach Transferschritten, gemäß denen kompatible Flüssigkeiten 6 zu pipettieren sind. Beim Analysieren der Transferschritte gemäß Fig. 12 wird die Steuerungseinrichtung 1 feststellen, dass gemäß den Transferschritten 1 und 8 zu vermischende Flüssigkeiten 6 und gemäß Transferschritt 15 die so gemischten Flüssigkeiten 6 zu pipettieren sind. Dies trifft entsprechend auch auf die Transferschritte 2, 9, 16 und 3, 10, 17 usw. zu. Diese Transferschritte mit kompatiblen Flüssigkeiten 6 sind in Fig. 12 mit der gleichen Schraffur dargestellt.

Beim Analysieren werden die spezifizierten Transferschritte von der Steuerungseinrichtung 1 außerdem dahingehend untersucht, ob diese Transferschritte unabhängig von anderen Transferschritten sind bzw. die Ausführung dieser Transferschritte in einer anderen als der spezifizierten Reihenfolge erfolgen darf. Im Beispiel gemäß Fig. 12 müssen die Transferschritte 1 und 8 vor Transferschritt 15 erfolgen. Entsprechend müssen die Transferschritte 2 und 9 vor Transferschritt 16 erfolgen usw.

Die Steuerungseinrichtung 1 ist nun so ausgebildet, dass sie nach der Analyse automatisch die Reihenfolge der Ausführung dieser analysierten Transferschritte verändert, und zwar so, dass Transferschritte, gemäß denen Flüssigkeit 6 in dieselbe Aufnahmeeinrichtung 5 abgegeben und die so entstandene Flüssigkeit 6 aus dieser Aufnahmeeinrichtung 5 aufgenommen und in eine andere Aufnahmeeinrichtung 5 abgegeben werden soll, unmittelbar aufeinanderfolgen. Die daraus resultierende geänderte Reihenfolge ist in Fig. 13 dargestellt und an der jeweiligen Ziffer in den Ziel-Wells 16'A bis 16'DDD in den Pipettiereinheit-Äquivalenten 18C und 18D erkennbar.

Z. B. ist der zuvor als achter Transferschritt spezifizierte Transferschritt nun als zweiter Transferschritt auszuführen. Der zuvor als 15. Transferschritt spezifizierte Transferschritt ist nun als dritter Transferschritt auszuführen. Der zuvor als siebter Transferschritt spezifizierte Transferschritt ist nun als 19. Transferschritt auszuführen. Der zuvor als 14. Transferschritt spezifizierte Transferschritt ist nun als 20. Transferschritt auszuführen. Der zuvor als 21. Transferschritt spezifizierte Transferschritt ist weiterhin als 21. Transferschritt auszuführen.

Durch die geänderte Reihenfolge der Ausführung der Transferschritte werden hier nur sieben statt 21 Wechsel von jeweils acht Pipettenspitzen 7 vorgenommen, nämlich nach den Transferschritten 3, 6, 9, 12, 15, 18 und 21. Auf diese Weise wird Material (Pipettenspitzen 7) und Zeit (für die Wege zum Abfallbehälter 32 und zurück) gespart. So werden hier statt 168 Pipettenspitzen 7 nur 56 Pipettenspitzen 7 gewechselt und weggeworfen.

Die zuvor beschriebenen Aspekte sind vorzugsweise untereinander kombinierbar. Ferner kann vorgesehen sein, dass die in Zusammenhang mit den Konfigurationsoberflächen 15B bis 15L erläuterten Prinzipien als unterschiedliche Optionen und/oder mittels eines oder mehrerer Kontextmenüs in derselben Konfigurationsoberfläche anwählbar sind. Hierzu können insbesondere virtuelle Schalter, Checkboxen, Drop-Down-Menüs, Soft-Buttons o. dgl. vorgesehen sein. Ferner ist bevorzugt, dass in derselben Konfigurationsoberfläche 15B bis 15L sowohl Wells 16A bis 16F auswählbar als auch das Kontextmenü generierbar und hierdurch oder in anderer Weise eine oder mehrere Optionen wählbar oder konfigurierbar sind, vorzugsweise wobei eine oder mehrere Volumendifferenz/-en, Volumina, Zielvolumina und/oder das Zusammenfassen, das Aufteilen oder eine geänderte Anordnung festlegbar, einstellbar oder aktivierbar ist/sind.

Die vorliegende Erfindung betrifft auch ein Verfahren zur Steuerung des Pipettierautomaten 2 mit der Steuerungseinrichtung 1, wobei die zuvor beschriebenen Prinzipien in Alleinstellung oder in Kombination durchgeführt werden. Vorzugsweise wird hierzu eine Eingabe mittels der Eingabeeinrichtung 10 durch die Steuerungseinrichtung 1 derart interpretiert, dass auswählbare Wells 16, 16' teilweise, einzeln oder gruppenweise ausgewählt werden, insbesondere durch Bewegen des Auswahlwerkzeugs 22 und einer währenddessen und/oder daraufhin folgenden Eingabe, beispielsweise mittels eines Schalters oder Knopfes. Die Steuerungseinrichtung 1 interpretiert dies vorzugsweise als Auswahl des jeweiligen Wells 16, 16' oder einer entsprechenden ersten oder zweiten Gruppe 27, 29 und führt vorzugsweise einzelne oder eine Kombination von Schritten aus, von denen zuvor beschrieben worden ist, dass die Steuerungseinrichtung 1 hierzu geeignet ist.

Ferner ist bevorzugt, dass das Verfahren die Steuerung des Pipettierautomaten 2 umfasst. Hierzu kann vorgesehen sein, dass die Steuerungseinrichtung 1 die unterschiedlichen Auswahlen von Wells 16, 16', besonders bevorzugt in der jeweiligen chronologischen Reihenfolge der Auswahl und unter Berücksichtigung der jeweils während der Auswahl aktiven Optionen, dadurch steuert, dass die Steuerungseinrichtung 1 Maschinenbefehle generiert und an den Pipettierautomaten 2 übermittelt. Hierdurch werden insbesondere Aktuatoren 3A bis 3C des Pipettierautomaten 2 derart gesteuert, dass die zuvor konfigurierten Aufnahmen und Abgaben der jeweiligen Transfervolumina aus bzw. in die Aufnahmeeinrichtungen 5 bewirkt werden.

### Bezugszeichenliste:

- 1: Steuerungseinrichtung
- 2: Pipettierautomat
- 3: Aktuator
- 4: Pipettiervorrichtung
- 5: Aufnahmeeinrichtung
- 6: Flüssigkeit
- 7: Pipettenspitze
- 8: Pipettiereinheit
- 9: Datenschnittstelle
- 10: Eingabeeinrichtung
- 11: Tastatur
- 12: Computermaus
- 13: Anzeigeeinrichtung
- 14: Speichermedium
- 15: Konfigurationsoberfläche
- 16: Well auf der 1. Seite
- 16': Well auf der 2. Seite
- 17: Virtuelle Position
- 18: Pipettiereinheit-Äquivalent
- 19: Umschaltmittel
- 20: Konfigurationsanzeige
- 21: Anzeigeabschnitt
- 22: Auswahlwerkzeug
- 23: Transferparameter
- 24: Pfeil
- 25: Ist-Volumen-Parameter
- 26: Eingabemaske
- 27: erste Gruppe
- 28: Minus-Symbol
- 29: zweite Gruppe
- 30: Plus-Symbol
- 31: Zielvolumen
- 32: Abfallbehälter
- 33: Abfallbehälter-Äquivalent
- 34: Pipettenspitzenhalter
- 35: Pipettenspitzenhalter-Äquivalent
- 36: Pipettenspitzen-Äquivalent

## Patentansprüche

1. Computer-implementierte Steuerungseinrichtung zum Steuern eines Pipettierautomaten (2), wobei
- die Steuerungseinrichtung (1) zum Steuern mindestens eines Aktuators (3) zur Bewegung einer Pipettiervorrichtung (4) zwischen Aufnahmeeinrichtungen (5) für zu pipettierende Flüssigkeiten (6) ausgebildet ist,
- die Steuerungseinrichtung (1) derart zum Steuern der Pipettiervorrichtung (4) ausgebildet ist, dass mittels der Pipettiervorrichtung (4) in einem Transferschritt ein bestimmtes Transfervolumen an Flüssigkeit (6) aus mindestens einer der Aufnahmeeinrichtungen (5) aufnehmbar und wenigstens ein Teil des Transfervolumens an Flüssigkeit (6) in mindestens eine andere der Aufnahmeeinrichtungen (5) abgebbar ist,
- die Steuerungseinrichtung (1) eine Eingabeeinrichtung (10) und eine Anzeigeeinrichtung (13) aufweist,
- durch die Anzeigeeinrichtung (10) mindestens eine Konfigurationsoberfläche (15) darstellbar ist,
- die Steuerungseinrichtung (1) dazu ausgebildet ist, dass in der Konfigurationsoberfläche (15) die Aufnahmeeinrichtungen (5) durch graphisch dargestellte Wells (16, 16') repräsentiert werden,
- die Aufnahmeeinrichtungen (5), aus denen zu pipettierende Flüssigkeit (6) durch die Pipettiervorrichtung (4) aufgenommen werden soll, mit Quell-Wells (16) und die Aufnahmeeinrichtungen (5), in die zu pipettierende Flüssigkeit (6) durch die Pipettiervorrichtung (4) abgegeben werden soll, mit Ziel-Wells (16') korrespondieren,
- mittels der Eingabeeinrichtung (10) in der Konfigurationsoberfläche (15) mehrere Quell-Wells (16) und mehrere Ziel-Wells (16') auswählbar sind,
- die Steuerungseinrichtung (1) dazu ausgebildet ist, dass zum Spezifizieren mehrerer Transferschritte und deren Reihenfolge der Ausführung durch die Pipettiervorrichtung (4) und den Aktuator (3) in der Konfigurationsoberfläche (15) mehrere zuvor ausgewählte Quell-Wells (16) mindestens einem Ziel-Well (16') durch Auswahl dieses Ziel-Wells (16') zuordenbar sind,
**dadurch gekennzeichnet,**
**dass** die Steuerungseinrichtung (1) dazu ausgebildet ist,
- nach dem Spezifizieren mehrerer Transferschritte und deren Reihenfolge der Ausführung durch die Pipettiervorrichtung (4) und den Aktuator (3) und vor der Ausführung dieser spezifizierten Transferschritte durch die Pipettiervorrichtung (4) und den Aktuator (3) die spezifizierte Reihenfolge der Ausführung dieser Transferschritte und die in diesen Transferschritten zu pipettierende Flüssigkeit (6) hinsichtlich des Materialverbrauchs und/oder der für die Ausführung der spezifizierten Transferschritte benötigten Zeit zu analysieren und
- nach der Analyse automatisch die Reihenfolge der Ausführung dieser Transferschritte zu verändern und/oder mehrere dieser Transferschritte zusammenzufassen, um den Materialverbrauch und/oder die für die Ausführung der spezifizierten Transferschritte benötigte Zeit zu verringern.

2. Steuerungseinrichtung nach Anspruch 1, **dadurch gekennzeichnet, dass** die Steuerungseinrichtung (1) zum Bestimmen von Transferschritten mit kompatiblen Flüssigkeiten (6) bei der Analyse der Transferschritte ausgebildet ist und, vorzugsweise, die Anzeigeeinrichtung (13) zur optisch unterscheidbaren Darstellung von Quell- und/oder Ziel-Wells (16, 16') mit kompatiblen Flüssigkeiten (6) in der Konfigurationsoberfläche (15) ausgebildet ist.

3. Steuerungseinrichtung nach Anspruch 1 oder 2, **dadurch gekennzeichnet, dass** die Steuerungseinrichtung (1) dazu ausgebildet ist, automatisch die Reihenfolge der Ausführung dieser Transferschritte so zu verändern, dass
- Transferschritte, gemäß denen Flüssigkeit (6) aus derselben Aufnahmeeinrichtung (5) aufgenommen oder in dieselbe Aufnahmeeinrichtung (5) abgegeben werden soll, unmittelbar aufeinanderfolgen, und, vorzugsweise, unmittelbar anschließend einen Wechsel einer Pipettenspitze (7) der Pipettiervorrichtung (4) zu veranlassen, und/oder
- Transferschritte, gemäß denen Flüssigkeit (6) in dieselbe Aufnahmeeinrichtung (5) abgegeben und die so entstandene Flüssigkeit (6) aus dieser Aufnahmeeinrichtung (5) aufgenommen und in eine andere Aufnahmeeinrichtung (5) abgegeben werden soll, unmittelbar aufeinanderfolgen, und, vorzugsweise, unmittelbar anschließend einen Wechsel einer Pipettenspitze (7) der Pipettiervorrichtung (4) zu veranlassen.

4. Steuerungseinrichtung nach einem der vorhergehenden Ansprüche, **dadurch gekennzeichnet, dass** die Steuerungseinrichtung (1) dazu ausgebildet ist, Transferschritte zusammenzufassen, indem
- Transferschritte, gemäß denen ein bestimmtes Transfervolumen an Flüssigkeit (6) aus derselben Aufnahmeeinrichtung (5) als Quelle aufgenommen und in bestimmte Aufnahmeeinrichtungen (5) als Ziele abgegeben werden soll, automatisch durch einen Transferschritt ersetzt werden, gemäß dem einmalig die Summe der bestimmten Transfervolumina an Flüssigkeit (6) aus der Quelle (5) aufgenommen und das jeweils bestimmte Transfervolumen an Flüssigkeit (6) in das jeweilige Ziel (5) abgegeben werden soll, und/oder
- Transferschritte, gemäß denen ein bestimmtes Transfervolumen an Flüssigkeit (6) aus bestimmten Aufnahmeeinrichtungen (5) als Quellen aufgenommen und in eine andere Aufnahmeeinrichtung (5) als dasselbe Ziel abgegeben werden soll, automatisch durch einen Transferschritt ersetzt werden, gemäß dem unmittelbar nacheinander das jeweils bestimmte Transfervolumen an Flüssigkeit (6) aus der jeweiligen Quelle (5) aufgenommen und einmalig die Summe der bestimmten Transfervolumina an Flüssigkeit (6) in das Ziel (5) abgegeben werden soll.

5. Steuerungseinrichtung nach einem der vorhergehenden Ansprüche, **dadurch gekennzeichnet, dass** die Anzeigeeinrichtung (13) zur Anzeige der veränderten Reihenfolge der Transferschritte und/oder eines geänderten Transfervolumens in der Konfigurationsoberfläche (15) und/oder zur Anzeige des Ablaufs der Transferschritte in einer weiteren Konfigurationsoberfläche (15) ausgebildet ist.

6. Steuerungseinrichtung nach einem der vorhergehenden Ansprüche, **dadurch gekennzeichnet, dass** die Steuerungseinrichtung (1) dazu ausgebildet ist, die Pipettiervorrichtung (4) und den Aktuator (3) so zu steuern, dass
- unmittelbar nach einem zusammengefassten Transferschritt und/oder
- nach einem Transferschritt mit einer Flüssigkeit (6) und vor einem nachfolgenden Transferschritt mit einer anderen Flüssigkeit (6)
ein Wechsel einer Pipettenspitze (7) der Pipettiervorrichtung (4) durchgeführt wird.

7. Steuerungseinrichtung nach einem der vorhergehenden Ansprüche, **dadurch gekennzeichnet, dass** vor oder nach der Analyse der Transferschritte mittels der Eingabeeinrichtung (10) in der Konfigurationsoberfläche (15) Transferschritte von der Analyse der Steuerungseinrichtung (1) ausschließbar oder mehrere unterschiedliche Flüssigkeiten (6) als zur Analyse der Transferschritte zu vermischen auswählbar sind.

8. Pipettierautomat mit einer Steuerungseinrichtung (1) gemäß einem der voranstehenden Ansprüche, wobei der Pipettierautomat (2) mindestens einen Aktuator (3) zur Bewegung einer Pipettiervorrichtung (4) zwischen Aufnahmeeinrichtungen (5) für zu pipettierende Flüssigkeiten (6) aufweist, wobei der Aktuator (3) durch die Steuerungseinrichtung (1) steuerbar ist.

9. Verfahren zur Steuerung eines Pipettierautomaten (2) zur Steuerung mindestens eines Aktuators (3) zur Bewegung einer Pipettiervorrichtung (2) zwischen Aufnahmeeinrichtungen (5) für zu pipettierende Flüssigkeiten (6), mittels einer Steuerungseinrichtung (1), wobei
- die Steuerungseinrichtung (1) derart zum Steuern der Pipettiervorrichtung (4) ausgebildet ist, dass mittels der Pipettiervorrichtung (4) in einem Transferschritt ein bestimmtes Transfervolumen an Flüssigkeit (6) aus mindestens einer der Aufnahmeeinrichtungen (5) aufnehmbar und wenigstens ein Teil des Transfervolumens an Flüssigkeit (6) in mindestens eine andere der Aufnahmeeinrichtungen (5) abgebbar ist,
- die Steuerungseinrichtung (1) eine Eingabeeinrichtung (10) und eine Anzeigeeinrichtung (13) aufweist,
- durch die Anzeigeeinrichtung (10) mindestens eine Konfigurationsoberfläche (15) dargestellt wird,
- in der Konfigurationsoberfläche (15) die Aufnahmeeinrichtungen (5) durch graphisch dargestellte Wells (16, 16') repräsentiert werden,
- die Aufnahmeeinrichtungen (5), aus denen zu pipettierende Flüssigkeit (6) durch die Pipettiervorrichtung (4) aufgenommen werden soll, mit Quell-Wells (16) und die Aufnahmeeinrichtungen (5), in die zu pipettierende Flüssigkeit (6) durch die Pipettiervorrichtung (4) abgegeben werden soll, mit Ziel-Wells (16') korrespondieren,
- mittels der Eingabeeinrichtung (10) in der Konfigurationsoberfläche (15) mehrere Quell-Wells (16) und mehrere Ziel-Wells (16') auswählbar sind,
- mehrere Transferschritte und deren Reihenfolge der Ausführung durch die Pipettiervorrichtung (4) und den Aktuator (3) spezifiziert werden, indem in der Konfigurationsoberfläche (15) mehrere zuvor ausgewählte Quell-Wells (16) mindestens einem Ziel-Well (16') durch Auswahl dieses Ziel-Wells (16') zugeordnet werden,
**dadurch gekennzeichnet,**
- **dass**, vor der Ausführung dieser spezifizierten Transferschritte durch die Pipettiervorrichtung (4) und den Aktuator (3), die Steuerungseinrichtung (1) die spezifizierte Reihenfolge der Ausführung dieser Transferschritte und die in diesen Transferschritten zu pipettierende Flüssigkeit (6) hinsichtlich des Materialverbrauchs und/oder der für die Ausführung der spezifizierten Transferschritte benötigten Zeit analysiert und
- **dass** die Steuerungseinrichtung (1) nach der Analyse automatisch die Reihenfolge der Ausführung dieser Transferschritte verändert und/oder mehrere dieser Transferschritte zusammenzufasst, um den Materialverbrauch und/oder die für die Ausführung der spezifizierten Transferschritte benötigte Zeit zu verringern.

10. Verfahren nach Anspruch 9, **dadurch gekennzeichnet, dass** die Steuerungseinrichtung (1) bei der Analyse Transferschritte mit kompatiblen Flüssigkeiten (6) bestimmt und, vorzugsweise, die Anzeigeeinrichtung (13) Quell- und/oder Ziel-Wells (16, 16') mit kompatiblen Flüssigkeiten (6) in der Konfigurationsoberfläche (15) optisch unterscheidbar darstellt.

11. Verfahren nach Anspruch 9 oder 10, **dadurch gekennzeichnet, dass** die Steuerungseinrichtung (1) automatisch die Reihenfolge der Ausführung dieser Transferschritte so verändert, dass
- Transferschritte, gemäß denen Flüssigkeit (6) aus derselben Aufnahmeeinrichtung (5) aufgenommen oder in dieselbe Aufnahmeeinrichtung (5) abgegeben werden soll, unmittelbar aufeinanderfolgen, und, vorzugsweise, unmittelbar anschließend ein Wechsel einer Pipettenspitze (7) der Pipettiervorrichtung (4) veranlasst wird und/oder
- Transferschritte, gemäß denen Flüssigkeit (6) in dieselbe Aufnahmeeinrichtung (5) abgegeben und die so entstandene Flüssigkeit (6) aus dieser Aufnahmeeinrichtung (5) aufgenommen und in eine andere Aufnahmeeinrichtung (5) abgegeben werden soll, unmittelbar aufeinanderfolgen und, vorzugsweise, unmittelbar anschließend ein Wechsel einer Pipettenspitze (7) der Pipettiervorrichtung (4) veranlasst wird.

12. Verfahren nach einem der Ansprüche 9 bis 11, **dadurch gekennzeichnet, dass** die Steuerungseinrichtung (1) Transferschritte zusammenzufasst, indem
- Transferschritte, gemäß denen ein bestimmtes Transfervolumen an Flüssigkeit (6) aus derselben Aufnahmeeinrichtung (5) als Quelle aufgenommen und in bestimmte Aufnahmeeinrichtungen (5) als Ziele abgegeben werden soll, automatisch durch einen Transferschritt ersetzt werden, gemäß dem einmalig die Summe der bestimmten Transfervolumina an Flüssigkeit (6) aus der Quelle (5) aufgenommen und das jeweils bestimmte Transfervolumen an Flüssigkeit (6) in das jeweilige Ziel (5) abgegeben werden soll und/oder
- Transferschritte, gemäß denen ein bestimmtes Transfervolumen an Flüssigkeit (6) aus bestimmten Aufnahmeeinrichtungen (5) als Quellen aufgenommen und in eine weitere Aufnahmeeinrichtung (5) als dasselbe Ziel (5) abgegeben werden soll, automatisch durch einen Transferschritt ersetzt werden, gemäß dem unmittelbar nacheinander das jeweils bestimmte Transfervolumen an Flüssigkeit (6) aus der jeweiligen Quelle (5) aufgenommen und einmalig die Summe der bestimmten Transfervolumina an Flüssigkeit (6) in das Ziel (5) abgegeben werden soll.

13. Verfahren nach einem der Ansprüche 9 bis 12, **dadurch gekennzeichnet, dass** die Anzeigeeinrichtung (13) die veränderte Reihenfolge der Transferschritte und/oder ein geändertes Transfervolumens in der Konfigurationsoberfläche (15) und/oder den Ablauf der Transferschritte in einer weiteren Konfigurationsoberfläche (15) anzeigt.

14. Verfahren nach einem der Ansprüche 9 bis 13, **dadurch gekennzeichnet, dass** die Steuerungseinrichtung (1) die Pipettiervorrichtung (4) und den Aktuator (3) so steuert, dass
- unmittelbar nach einem zusammengefassten Transferschritt und/oder
- nach einem Transferschritt mit einer Flüssigkeit (6) und vor einem nachfolgenden Transferschritt mit einer anderen Flüssigkeit (6)
ein Wechsel einer Pipettenspitze (7) der Pipettiervorrichtung (4) durchgeführt wird.

15. Verfahren nach einem der Ansprüche 9 bis 14, **dadurch gekennzeichnet, dass** vor oder nach der Analyse der Transferschritte mittels der Eingabeeinrichtung (10) in der Konfigurationsoberfläche (15) Transferschritte von der Analyse der Steuerungseinrichtung (1) ausgeschlossen oder mehrere unterschiedliche Flüssigkeiten (6) als zur Analyse der Transferschritte zu vermischen ausgewählt werden und/oder dass eine bereits benutzte und von verschiedenen Arten von Flüssigkeit (6) kontaminierte Pipettenspitze (7) der Pipettiervorrichtung (4) wiederverwendet wird, insbesondere zur nochmaligen Benutzung für mindestens einen weiteren Transferschritt.

16. Computerprogrammprodukt mit Programmcodemitteln, die dazu ausgebildet sind, das Verfahren gemäß einem der Ansprüche 9 bis 15 auszuführen, wenn die Programmcodemittel auf einem Computer oder durch einen Prozessor ausgeführt werden.

17. Verwendung einer Steuerungseinrichtung (1) gemäß einem der Ansprüche 1 bis 7 zur Steuerung eines Pipettierautomaten (2) gemäß Anspruch 8.

## Claims

1. Computer-implemented control device for controlling an automatic pipetting machine (2), wherein
- the control device (1) is designed to control at least one actuator (3) for moving a pipetting device (4) between receptacle devices (5) for liquids (6) to be pipetted,
- the control device (1) is designed to control the pipetting device (4) in such a way that a specific transfer volume of liquid (6) can be taken up from at least one of the receptacle devices (5) by means of the pipetting device (4) in a transfer step and at least part of the transfer volume of liquid (6) can be dispensed into at least one other of the receptacle devices (5),
- the control device (1) has an input device (10) and a display device (13),
- at least one configuration interface (15) can be displayed by the display device (10),
- the control device (1) is designed so that the receptacle devices (5) are represented in the configuration interface (15) by graphically depicted wells (16, 16'),
- the receptacle devices (5), from which liquid (6) to be pipetted is to be taken up by the pipetting device (4), correspond to source wells (16) and the receptacle devices (5), into which liquid (6) to be pipetted is to be dispensed by the pipetting device (4), correspond to target wells (16'),
- several source wells (16) and several target wells (16') can be selected in the configuration interface (15) by means of the input device (10),
- the control device (1) is designed such that, in order to specify several transfer steps and their order of execution by the pipetting device (4) and the actuator (3), in the configuration interface (15) multiple previously selected source wells (16) can be assigned to at least one target well (16') by selecting this target well (16'),
**characterized in that**
the control device (1) is designed so as
- to analyze, after specifying several transfer steps and their order of execution by the pipetting device (4) and the actuator (3) and before the execution of these specified transfer steps by the pipetting device (4) and the actuator (3), the specified order of execution of these transfer steps and the liquid (6) to be pipetted in these transfer steps with regard to the material consumption and/or the time required for the execution of the specified transfer steps, and
- to automatically change the order of execution of these transfer steps after the analysis and/or combine several of these transfer steps in order to reduce the material consumption and/or the time required for the execution of the specified transfer steps.

2. The control device according to claim 1, **characterized in that** the control device (1) is designed to determine transfer steps with compatible liquids (6) during the analysis of the transfer steps and, preferably, the display device (13) is designed for the optically distinguishable display of source and/or target wells (16, 16') with compatible liquids (6) in the configuration interface (15).

3. The control device according to claim 1 or 2, **characterized in that** the control device (1) is designed to automatically change the order of execution of these transfer steps in such a way that
- transfer steps, according to which liquid (6) is to be taken up from the same receptacle device (5) or dispensed into the same receptacle device (5), immediately follow one another, and, preferably, immediately afterwards a change of a pipette tip (7) of the pipetting device (4) is initiated, and/or
- transfer steps, according to which liquid (6) is to be dispensed into the same receptacle device (5) and the liquid (6) resulting therefrom is to be taken up from this receptacle device (5) and dispensed into another receptacle device (5), are immediately successive, and, preferably, immediately afterwards a change of a pipette tip (7) of the pipetting device (4) is initiated.

4. The control device according to one of the preceding claims, **characterized in that** the control device (1) is designed to combine transfer steps by
- automatically replacing transfer steps, according to which a specific transfer volume of liquid (6) is to be taken up from the same receptacle device (5) as source and dispensed into specific receptacle devices (5) as target, by a transfer step, according to which the sum of the specific transfer volumes of liquid (6) is to be taken up once from the source (5) and the respective specific transfer volume of liquid (6) is to be dispensed into the respective target (5), and/or
- automatically replacing transfer steps, according to which a specific transfer volume of liquid (6) is to be taken up from specific receptacle devices (5) as sources and dispensed into another receptacle device (5) as the same target, by a transfer step, according to which the specific transfer volume of liquid (6) is to be taken up from the respective source (5) immediately successively and the sum of the specific transfer volumes of liquid (6) is to be dispensed once into the target (5).

5. The control device according to one of the preceding claims, **characterized in that** the display device (13) is designed to display the changed order of the transfer steps and/or a changed transfer volume in the configuration interface (15) and/or to display the order of the transfer steps in a further configuration interface (15).

6. The control device according to one of the preceding claims, **characterized in that** the control device (1) is designed to control the pipetting device (4) and the actuator (3) in such a way that
- immediately after a combined transfer step and/or
- after a transfer step with a liquid (6) and before a subsequent transfer step with another liquid (6)
a pipette tip (7) of the pipetting device (4) is changed.

7. The control device according to one of the preceding claims, **characterized in that**, before or after the analysis of the transfer steps by means of the input device (10) in the configuration interface (15), transfer steps can be excluded from the analysis of the control device (1) or several different liquids (6) can be selected as being to be mixed for the analysis of the transfer steps.

8. Automatic pipetting machine with a control device (1) according to one of the preceding claims, wherein the automatic pipetting machine (2) has at least one actuator (3) for moving a pipetting device (4) between receptacle devices (5) for liquids (6) to be pipetted, wherein the actuator (3) can be controlled by the control device (1).

9. Method for controlling an automatic pipetting device (2) for controlling at least one actuator (3) for moving a pipetting device (2) between receptacle devices (5) for liquids (6) to be pipetted, by means of a control device (1), wherein
- the control device (1) is designed to control the pipetting device (4) in such a way that a specific transfer volume of liquid (6) can be taken from at least one of the receptacle devices (5) by means of the pipetting device (4) in a transfer step and at least part of the transfer volume of liquid (6) can be dispensed into at least one other of the receptacle devices (5),
- the control device (1) has an input device (10) and a display device (13),
- at least one configuration interface (15) is displayed by the display device (10),
- in the configuration interface (15), the receptacle devices (5) are represented by graphically depicted wells (16, 16'),
- the receptacle devices (5), from which liquid (6) to be pipetted is to be taken up by the pipetting device (4), correspond to source wells (16) and the receptacle devices (5), into which liquid (6) to be pipetted is to be dispensed by the pipetting device (4), correspond to target wells (16'),
- several source wells (16) and several target wells (16') can be selected in the configuration interface (15) by means of the input device (10),
- several transfer steps and their order of execution by the pipetting device (4) and the actuator (3) are specified in the configuration interface (15) by assigning multiple previously selected source wells (16) to at least one target well (16') by selecting this target well (16'),
**characterized in that**
- prior to the execution of these specified transfer steps by the pipetting device (4) and the actuator (3), the control device (1) analyzes the specified order of execution of these transfer steps and the liquid (6) to be pipetted in these transfer steps with regard to the material consumption and/or the time required for the execution of the specified transfer steps and
- the control device (1) automatically changes the order of execution of these transfer steps after the analysis and/or combines several of these transfer steps, in order to reduce the material consumption and/or the time required for the execution of the specified transfer steps.

10. The method according to claim 9, **characterized in that** the control device (1) determines transfer steps with compatible liquids (6) during the analysis and, preferably, the display device (13) displays source and/or target wells (16, 16') with compatible liquids (6) in the configuration interface (15) in an optically distinguishable manner.

11. The method according to claim 9 or 10, **characterized in that** the control device (1) automatically changes the order of execution of these transfer steps in such a way that
- transfer steps, according to which liquid (6) is to be taken up from the same receptacle device (5) or dispensed into the same receptacle device (5), are immediately successive, and, preferably, immediately afterwards a change of a pipette tip (7) of the pipetting device (4) is initiated and/or
- transfer steps, according to which liquid (6) is to be dispensed into the same receptacle device (5) and the liquid (6) resulting therefrom is to be taken up from this receptacle device (5) and dispensed into another receptacle device (5), are immediately successive and, preferably, immediately afterwards a change of a pipette tip (7) of the pipetting device (4) is initiated.

12. The method according to one of claims 9 to 11, **characterized in that** the control device (1) combines transfer steps by
- automatically replacing transfer steps, according to which a specific transfer volume of liquid (6) is to be taken up from the same receptacle device (5) as source and dispensed into specific receptacle devices (5) as targets, by a transfer step, according to which the sum of the specific transfer volumes of liquid (6) is to be taken up once from the source (5) and the respective specific transfer volume of liquid (6) is to be dispensed into the respective target (5), and/or
- automatically replacing transfer steps, according to which a specific transfer volume of liquid (6) is to be taken up from specific receptacle devices (5) as sources and dispensed into a further receptacle device (5) as the same target (5), by a transfer step, according to which the specific transfer volume of liquid (6) is to be taken up from the respective source (5) immediately successively and the sum of the specific transfer volumes of liquid (6) is to be dispensed once into the target (5).

13. The method according to one of claims 9 to 12, **characterized in that** the display device (13) displays the changed order of the transfer steps and/or a changed transfer volume in the configuration interface (15) and/or the order of the transfer steps in a further configuration interface (15).

14. The method according to one of claims 9 to 13, **characterized in that** the control device (1) controls the pipetting device (4) and the actuator (3) in such a way that
- immediately after a combined transfer step and/or
- after a transfer step with a liquid (6) and before a subsequent transfer step with another liquid (6)
a pipette tip (7) of the pipetting device (4) is changed.

15. The method according to one of claims 9 to 14, **characterized in that** before or after the analysis of the transfer steps by means of the input device (10) in the configuration interface (15) transfer steps are excluded from the analysis of the control device (1) or several different liquids (6) are selected as to be mixed for the analysis of the transfer steps and/or that an already used pipette tip (7) of the pipetting device (4) contaminated by different types of liquid (6) is reused, in particular for repeated use for at least one further transfer step.

16. A computer program product comprising program code means adapted to carry out the method according to any one of claims 9 to 15 when the program code means is executed on a computer or by a processor.

17. Use of a control device (1) according to one of claims 1 to 7 for controlling an automatic pipetting machine (2) according to claim 8.

## Revendications

1. Dispositif de commande mis en oeuvre par ordinateur pour commander un automate de pipetage (2), dans lequel
- le dispositif de commande (1) est conçu pour commander au moins un actionneur (3) pour déplacer un dispositif de pipetage (4) entre des dispositifs de réception (5) pour des liquides à pipeter (6),
- le dispositif de commande (1) est conçu pour commander le dispositif de pipetage (4) de telle sorte qu'au moyen du dispositif de pipetage (4), dans une étape de transfert, un volume de transfert déterminé de liquide (6) peut être prélevé dans au moins l'un des dispositifs de réception (5) et au moins une partie du volume de transfert de liquide (6) peut être délivrée dans au moins un autre des dispositifs de réception (5),
- le dispositif de commande (1) présente un dispositif d'entrée (10) et un dispositif d'affichage (13),
- au moins une interface de configuration (15) peut être représentée par le dispositif d'affichage (10),
- le dispositif de commande (1) est conçu pour que, dans l'interface de configuration (15), les dispositifs de réception (5) soient représentés par des puits (16, 16') représentés graphiquement,
- les dispositifs de réception (5), à partir desquels le liquide à pipeter (6) doit être prélevé par le dispositif de pipetage (4), correspondent à des puits de source (16) et les dispositifs de réception (5), dans lesquels le liquide à pipeter (6) doit être délivré par le dispositif de pipetage (4), correspondent à des puits de destination (16'),
- plusieurs ondes sources (16) et plusieurs ondes cibles (16') peuvent être sélectionnées dans l'interface de configuration (15) au moyen du dispositif de saisie (10),
- le dispositif de commande (1) est conçu de telle sorte que, pour spécifier plusieurs étapes de transfert et leur ordre d'exécution par le dispositif de pipetage (4) et l'actionneur (3) dans l'interface de configuration (15), plusieurs ondulations sources (16) préalablement sélectionnées peuvent être affectées à au moins une ondulation cible (16') par sélection de cette ondulation cible (16'),
**caractérisé en ce que**
**que** le dispositif de commande (1) est conçu,
- à analyser, après avoir spécifié plusieurs étapes de transfert et leur ordre d'exécution par le dispositif de pipetage (4) et l'actionneur (3) et avant l'exécution de ces étapes de transfert spécifiées par le dispositif de pipetage (4) et l'actionneur (3), l'ordre spécifié d'exécution de ces étapes de transfert et à analyser le liquide (6) à pipeter dans ces étapes de transfert en ce qui concerne la consommation de matière et/ou le temps nécessaire pour l'exécution des étapes de transfert spécifiées, et
- à modifier automatiquement, après analyse, l'ordre d'exécution de ces étapes de transfert et/ou à regrouper plusieurs de ces étapes de transfert afin de réduire la consommation de matière et/ou le temps nécessaire à l'exécution des étapes de transfert spécifiées.

2. Dispositif de commande selon la revendication 1, **caractérisé en ce que** le dispositif de commande (1) est conçu pour déterminer des étapes de transfert avec des liquides compatibles (6) lors de l'analyse des étapes de transfert et, de préférence, le dispositif d'affichage (13) est conçu pour représenter de manière optiquement distincte des puits source et/ou cible (16, 16') avec des liquides compatibles (6) dans l'interface de configuration (15).

3. Dispositif de commande selon la revendication 1 ou 2, **caractérisé en ce que** le dispositif de commande (1) est conçu pour modifier automatiquement l'ordre d'exécution de ces étapes de transfert de telle sorte que
- des étapes de transfert, selon lesquelles du liquide (6) doit être prélevé dans le même dispositif de réception (5) ou délivré dans le même dispositif de réception (5), se succèdent immédiatement, et, de préférence, à provoquer immédiatement après un changement d'une pointe de pipette (7) du dispositif de pipetage (4), et/ou
- étapes de transfert, selon lesquelles du liquide (6) doit être délivré dans le même dispositif de réception (5) et le liquide (6) ainsi obtenu doit être prélevé dans ce dispositif de réception (5) et délivré dans un autre dispositif de réception (5), se succèdent immédiatement, et, de préférence, provoquer immédiatement après un changement d'une pointe de pipette (7) du dispositif de pipetage (4).

4. Dispositif de commande selon l'une des revendications précédentes, **caractérisé en ce que** le dispositif de commande (1) est conçu pour regrouper des étapes de transfert, **en ce que**
- des étapes de transfert, selon lesquelles un volume de transfert déterminé de liquide (6) doit être prélevé du même dispositif de réception (5) en tant que source et délivré dans des dispositifs de réception (5) déterminés en tant que destinations, sont remplacées automatiquement par une étape de transfert, selon laquelle la somme des volumes de transfert déterminés de liquide (6) doit être prélevée une seule fois à partir de la source (5) et le volume de transfert déterminé respectif de liquide (6) doit être délivré dans la destination (5) respective, et/ou
- des étapes de transfert, selon lesquelles un volume de transfert déterminé de liquide (6) doit être prélevé dans des dispositifs de réception (5) déterminés en tant que sources et doit être délivré dans un autre dispositif de réception (5) en tant que même destination, sont remplacées automatiquement par une étape de transfert, selon laquelle le volume de transfert déterminé respectif de liquide (6) doit être prélevé directement l'un après l'autre dans la source (5) respective et la somme des volumes de transfert déterminés de liquide (6) doit être délivrée une fois dans la destination (5).

5. Dispositif de commande selon l'une des revendications précédentes, **caractérisé en ce que** le dispositif d'affichage (13) est conçu pour afficher l'ordre modifié des étapes de transfert et/ou un volume de transfert modifié dans l'interface de configuration (15) et/ou pour afficher le déroulement des étapes de transfert dans une autre interface de configuration (15).

6. Dispositif de commande selon l'une des revendications précédentes, **caractérisé en ce que** le dispositif de commande (1) est conçu pour commander le dispositif de pipetage (4) et l'actionneur (3) de telle sorte que
- immédiatement après une étape de transfert groupé et/ou
- après une étape de transfert avec un liquide (6) et avant une étape de transfert ultérieure avec un autre liquide (6)
un changement d'une pointe de pipette (7) du dispositif de pipetage (4) est effectué.

7. Dispositif de commande selon l'une des revendications précédentes, **caractérisé en ce qu'**avant ou après l'analyse des étapes de transfert au moyen du dispositif de saisie (10), des étapes de transfert peuvent être exclues de l'analyse du dispositif de commande (1) dans l'interface de configuration (15) ou plusieurs liquides différents (6) peuvent être sélectionnés comme devant être mélangés pour l'analyse des étapes de transfert.

8. Automate de pipetage avec un dispositif de commande (1) selon l'une des revendications précédentes, l'automate de pipetage (2) présentant au moins un actionneur (3) pour déplacer un dispositif de pipetage (4) entre des dispositifs de réception (5) pour des liquides à pipeter (6), l'actionneur (3) pouvant être commandé par le dispositif de commande (1).

9. Procédé de commande d'un automate de pipetage (2) pour la commande d'au moins un actionneur (3) pour le déplacement d'un dispositif de pipetage (2) entre des dispositifs de réception (5) pour des liquides à pipeter (6), au moyen d'un dispositif de commande (1), dans lequel
- le dispositif de commande (1) est conçu pour commander le dispositif de pipetage (4) de telle sorte qu'au moyen du dispositif de pipetage (4), dans une étape de transfert, un volume de transfert déterminé de liquide (6) peut être prélevé dans au moins l'un des dispositifs de réception (5) et au moins une partie du volume de transfert de liquide (6) peut être délivrée dans au moins un autre des dispositifs de réception (5),
- le dispositif de commande (1) présente un dispositif d'entrée (10) et un dispositif d'affichage (13),
- au moins une interface de configuration (15) est représentée par le dispositif d'affichage (10),
- dans l'interface de configuration (15), les dispositifs de prise de vue (5) sont représentés par des puits (16, 16') représentés graphiquement,
- les dispositifs de réception (5), à partir desquels le liquide à pipeter (6) doit être prélevé par le dispositif de pipetage (4), correspondent à des puits de source (16) et les dispositifs de réception (5), dans lesquels le liquide à pipeter (6) doit être délivré par le dispositif de pipetage (4), correspondent à des puits de destination (16'),
- plusieurs ondes sources (16) et plusieurs ondes cibles (16') peuvent être sélectionnées dans l'interface de configuration (15) au moyen du dispositif de saisie (10),
- plusieurs étapes de transfert et leur ordre d'exécution par le dispositif de pipetage (4) et l'actionneur (3) sont spécifiés en associant, dans l'interface de configuration (15), plusieurs puits sources (16) préalablement sélectionnés à au moins un puits cible (16') par sélection de ce puits cible (16'),
**caractérisé en ce que**
- avant l'exécution de ces étapes de transfert spécifiées par le dispositif de pipetage (4) et l'actionneur (3), le dispositif de commande (1) analyse l'ordre spécifié d'exécution de ces étapes de transfert et le liquide (6) à pipetter dans ces étapes de transfert en ce qui concerne la consommation de matière et/ou le temps nécessaire pour l'exécution des étapes de transfert spécifiées et
- le dispositif de commande (1) modifie automatiquement, après l'analyse, l'ordre d'exécution de ces étapes de transfert et/ou regroupe plusieurs de ces étapes de transfert, afin de réduire la consommation de matière et/ou le temps nécessaire à l'exécution des étapes de transfert spécifiées .

10. Procédé selon la revendication 9, **caractérisé en ce que** le dispositif de commande (1) détermine, lors de l'analyse, des étapes de transfert avec des liquides compatibles (6) et, de préférence, le dispositif d'affichage (13) représente des puits source et/ou cible (16, 16') avec des liquides compatibles (6) dans l'interface de configuration (15) de manière à pouvoir les distinguer visuellement.

11. Procédé selon la revendication 9 ou 10, **caractérisé en ce que** le dispositif de commande (1) modifie automatiquement l'ordre d'exécution de ces étapes de transfert de telle sorte que
- des étapes de transfert, selon lesquelles du liquide (6) doit être prélevé dans le même dispositif de réception (5) ou délivré dans le même dispositif de réception (5), se succèdent immédiatement, et, de préférence, un changement d'une pointe de pipette (7) du dispositif de pipetage (4) est provoqué immédiatement après, et/ou
- des étapes de transfert, selon lesquelles du liquide (6) doit être délivré dans le même dispositif de réception (5) et le liquide (6) ainsi obtenu doit être prélevé de ce dispositif de réception (5) et délivré dans un autre dispositif de réception (5), se succèdent immédiatement et, de préférence, un changement d'une pointe de pipette (7) du dispositif de pipetage (4) est provoqué immédiatement après.

12. Procédé selon l'une des revendications 9 à 11, **caractérisé en ce que** le dispositif de commande (1) regroupe des étapes de transfert en
- des étapes de transfert, selon lesquelles un volume de transfert déterminé de liquide (6) doit être prélevé du même dispositif de réception (5) en tant que source et délivré dans des dispositifs de réception (5) déterminés en tant que destinations, sont remplacées automatiquement par une étape de transfert, selon laquelle la somme des volumes de transfert déterminés de liquide (6) doit être prélevée une seule fois à partir de la source (5) et le volume de transfert déterminé respectif de liquide (6) doit être délivré dans la destination (5) respective et/ou
- des étapes de transfert, selon lesquelles un volume de transfert déterminé de liquide (6) doit être prélevé dans des dispositifs de réception (5) déterminés en tant que sources et doit être délivré dans un autre dispositif de réception (5) en tant que même destination (5), sont remplacées automatiquement par une étape de transfert, selon laquelle le volume de transfert respectivement déterminé de liquide (6) doit être prélevé directement l'un après l'autre dans la source (5) respective et la somme des volumes de transfert déterminés de liquide (6) doit être délivrée une fois dans la destination (5).

13. Procédé selon l'une des revendications 9 à 12, **caractérisé en ce que** le dispositif d'affichage (13) affiche l'ordre modifié des étapes de transfert et/ou un volume de transfert modifié dans l'interface de configuration (15) et/ou le déroulement des étapes de transfert dans une autre interface de configuration (15).

14. Procédé selon l'une des revendications 9 à 13, **caractérisé en ce que** le dispositif de commande (1) commande le dispositif de pipetage (4) et l'actionneur (3) de telle sorte que
- immédiatement après une étape de transfert groupé et/ou
- après une étape de transfert avec un liquide (6) et avant une étape de transfert ultérieure avec un autre liquide (6)
un changement d'une pointe de pipette (7) du dispositif de pipetage (4) est effectué.

15. Procédé selon l'une des revendications 9 à 14, **caractérisé en ce que**, avant ou après l'analyse des étapes de transfert au moyen du dispositif d'entrée (10) dans l'interface de configuration (15), des étapes de transfert sont exclues de l'analyse du dispositif de commande (1) ou plusieurs liquides différents (6) sont sélectionnés comme devant être mélangés pour l'analyse des étapes de transfert et/ou **en ce qu'**une pointe de pipette (7) du dispositif de pipetage (4) déjà utilisée et contaminée par différents types de liquide (6) est réutilisée, en particulier pour une nouvelle utilisation pour au moins une autre étape de transfert.

16. Produit programme d'ordinateur comprenant des moyens de code de programme adaptés pour mettre en oeuvre le procédé selon l'une quelconque des revendications 9 à 15, lorsque les moyens de code de programme sont exécutés sur un ordinateur ou par un processeur.

17. Utilisation d'un dispositif de commande (1) selon l'une des revendications 1 à 7 pour la commande d'un automate de pipetage (2) selon la revendication 8.
